# EUROPÄISCHE PATENTSCHRIFT

(11) **EP 0 499 823 B1**
(45) Veröffentlichungstag und Bekanntmachung des Hinweises auf die Patenterteilung: **05.06.1996**
(21) Anmeldenummer: 92101121.9
(22) Anmeldetag: 24.01.1992
(51) Int. Cl.: C07C 251/48, A01N 37/50, C07C 251/50, C07C 251/52, C07C 251/54, C07C 251/56, C07C 251/58, C07D 333/28, C07D 263/32, C07C 69/734, C07C 69/653

(54) **Imino-substituierte Phenylderivate, ihre Herstellung und diese enthaltende Fungizide**
Imino-substituted phenyl derivatives, their preparation and fungicides containing them
Dérivés de phényle imino substitués, leur preparation et fongicides les contenant

(30) Priorität: 20.02.1991 DE 4105160
(43) Veröffentlichungstag der Anmeldung: 26.08.1992
(60) Teilanmeldung: 95112355.3
(73) Patentinhaber: BASF Aktiengesellschaft, D-67063 Ludwigshafen (DE)
(72) Erfinder: Grammenos, Wassilios, Dr., W-6700 Ludwigshafen (DE); Harreus, Albrecht, Dr., W-6700 Ludwigshafen (DE); Sauter, Hubert, Dr., W-6800 Mannheim 1 (DE); Hellendahl, Beate, Dr., W-6800 Mannheim 24 (DE); Doetzer, Reinhard, Dr., W-6940 Weinheim (DE); Ammermann, Eberhard, Dr., W-6700 Ludwigshafen (DE); Lorenz, Gisela, Dr., W-6730 Neustadt (DE)

(56) Entgegenhaltungen:
- EP-A- 0 254 426
- EP-A- 0 331 061
- EP-A- 0 370 629
- EP-A- 0 415 569

## Beschreibung

Die vorliegende Erfindung betrifft neue substituierte Oximether, Hydrazone und Fungizide, welche diese Verbindungen enthalten.

Es ist bekannt, Phenylacrylsäureenolether als Fungizide (EP 370 629) zu verwenden. Ihre Wirkung ist jedoch unbefriedigend.

Es wurde nun überraschend gefunden, daß substituierte Oximether und Hydrazone der allgemeinen Formel I 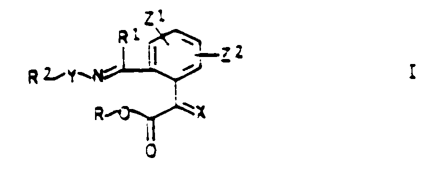
in der die Substituenten folgende Bedeutung haben:
- X: NOH oder NO-C₁-C₄-alkyl;
- R: Wasserstoff oder C₁-C₆-Alkyl;
- R¹: Wasserstoff;
- Z¹,Z²: gleich oder verschieden sind und Wasserstoff, Cyano, Nitro, Halogen, C₁-C₆-Alkyl, C₁-C₄-Halogenalkyl, C₁-C₄-Alkoxy, C₁-C₄-Halogenalkoxy, C₂-C₄-Alkenyl, C₂-C₄-Alkenyloxy, C₂-C₄-Halogenalkenyloxy oder C₂-C₄-Alkinyl bedeuten;
- Y: Sauerstoff (-O-), Stickstoff (-NH-) oder ein durch C₁-C₆-Alkyl (R³) substituierter Stickstoff (-NR³-);
- R²: Wasserstoff,
ggf. subst. Alkyl, Cycloalkyl, Alkenyl, Alkinyl oder Cycloalkenyl,
ggf. subst. Aryl, Arylalkyl, Aryloxyalkyl, Arylalkenyl oder Arylcarbonyl,
ggf. subst. Heteroaryl, Heteroarylalkyl, Heteroaryloxyalkyl, Heteroarylalkenyl oder Heteroarylcarbonyl,
ggf. subst. Heterocyclyl oder Heterocyclyloxy,
ggf. subst. Acyl oder
C₁-C₄-Alkoxycarbonyl-C₁-C₄-alkyl,
wobei Y und R³ einen ggf. subst. Ring bilden können,
eine gute fungizide Wirkung haben.

Außerdem wurde gefunden, daß die Verbindungen der Formel I eine gute insektizide, nematizide und akarizide Wirkung haben. Die fungizide Wirkung wird bevorzugt.

Im Hinblick auf ihre biologische Wirksamkeit zur Bekämpfung von Schadpilzen kommen Verbindungen I in Betracht, in denen die Substituenten z.B. die folgende Bedeutung haben:
X
C₁-C₄-Alkoxyimino wie Methoxy-, Ethoxy-, n- oder iso-Propoxy, n-, iso-, sec- oder tert.-Butoxyimino;
Z¹, Z²
Halogen wie Fluor, Chlor, Brom und Iod oder z.B. Methyl, Methoxy, Cyano und Nitro;
R, R³
Wasserstoff;
C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
R¹
Wasserstoff;
R²
Wasserstoff;
C₁-C₆-Alkyl wie vorstehend genannt;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
ggf. subst. C₃-C₁₅-Alkenyl, besonders C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Diemthyl-2-propenyl, 1,2-Diemthyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl,
2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl;
ggf. subst. C₃-C₈-Alkinyl, besonders C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
ggf. subst. C₃-C₈-Cycloalkyl wie Cyclopropyl, Cyclobutyl, Cyclopentyl, Cyclohexyl, Cycloheptyl und Cyclooctyl;
C₁-C₄-Alkoxycarbonyl-C₁-C₄-Alkyl wie Methoxycarbonylmethyl, Ethoxycarbonylmethyl, Propyloxycarbonylmethyl, 1-Methylethyloxycarbonylmethyl, Butyloxycarbonylmethyl, 1-Methylpropyloxycarbonylmethyl und 1,1-Dimethylethyloxycarbonylmethyl;
ggf. subst. C₅-C₈-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, Cyclooct-1-enyl, Cyclooct-2-enyl, Cyclooct-3-enyl und Cyclooct-1-enyl;
ggf. subst. Aryl (z.B. Phenyl, Naphthyl, Anthryl);
ggf. subst. Aryl-C₁-C₄-alkyl (z.B. Benzyl, 1-Phenethyl, 2-Phenethyl, 1-Phenylpropyl, 2-Phenylpropyl, 3-Phenpropyl, 2-Methyl-3-Phenylpropyl, 2-Methyl-2-Phenylpropyl, 4-Phenylbutyl);
ggf. subst. Acyl, z.B. C₁-C₄-Alkylcarbonyl wie Acetyl, Propionyl, Butyryl, Phenylacetyl und Chloracetyl;
ggf. subst. Arylcarbonyl wie Benzoyl und 2,4-Dichlorbenzoyl;
ggf. subst. Heteroarylcarbonyl wie 2-Pyridylcarbonyl;
ggf. subst. Aryl-C₁-C₄-alkenyl (z.B. Phenyl-1-ethenyl, 2-Phenyl-1-propenyl, 2,2-Diphenylethenyl, 1-Phenyl-1-propen-2-yl und 1-Phenylbutenyl);
ggf. subst. Aryloxy-C₁-C₄-Alkyl wie Phenoxymethyl, Phenoxyethyl und Phenoxypropyl;
ggf. subst. Heteroaryl (z.B. 2-Pyridyl, 3-Pyridyl, 4-Pyridyl, 3-Pyridazinyl, 4-Pyridazinyl, 1-Pyrazolyl, 3-Pyrazolyl, 4-Pyrazolyl, 2-Pyrazinyl, 2-Pyrimidinyl, 4-Pyrimidinyl, 5-Pyrimidinyl, 1-Indazolyl, 3-Indazolyl, 2-Furyl, 3-Furyl, 1-Pyrrolyl, 2-Pyrrolyl, 3-Pyrrolyl, 2-Thiophenyl, 3-Thiophenyl, 1-Imidazolyl, 2-Imidazolyl, 4-Imidazolyl, 5-Imidazolyl, 2-Isoindolyl, 1-Indolyl, 1-Indazolyl, 1,2-Benzothiazol-3-yl, 1,3-Benzothiazol-2-yl, 3-Isoaxazolyl, 4-Isoocazolyl, 5-Isoazolyl, 3-Isothiazolyl, 4-Isothiazolyl, 5-Isothiazolyl, 1,2-Benzooxazol-3-yl, 1,3-Benzoxazol-2-yl, 1,2,4-Triazolyl, 1,3,4-Triazolyl, 7-Purinyl, 2-Chinonyl, 3-Chinonyl, 4-Chinonyl, 1-Isochinolyl, 3-Isochinolyl, 4-Isochinolyl, 2-Benzofuranyl, 3-Benzofuranyl, 1-Isobenzofuranyl, 1,2,4-Triazin-3-yl und 1,3,5-Triazin-2-yl;
ggf. subst. Heteroaryl-C₁-C₄-alkyl (z.B. 2-Pyridylmethyl, 3-Pyridylmethyl);
ggf. subst. Heteroaryl-C₂-C₄-alkenyl (z.B. 2'-Furyl-2-ethenyl, 2'-Thienyl-2-ethenyl, 3'-Pyridyl-2-ethenyl);
ggf. subst. Heterocyclyl (z.B. Oxiranyl, 1-Aziridinyl, 1-Azetidinyl, 1-Pyrrolidinyl, 2-Tetrahydrofuryl, 2-Tetrahydropyranyl, 3-Tetrahydropyranyl, 1-Piperidinyl, 1-Morpholinyl, 1-Piperazinyl, 1,3-Dioxanyl, 3-Tetrahydrothiopyranyl);
Die mit "ggf. substituiert" bezeichneten Reste enthalten neben Wasserstoff z.B. die folgenden Reste:
Halogen wie Fluor, Chlor, Brom und Iod;
C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
C₁-C₆-Alkyloxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 1,1-Dimethylpropyloxy, 1,2-Dimethylpropyloxy, 2,2-Dimethylpropyloxy, 1-Ethylpropyloxy, Hexyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethylbutyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
C₁-C₄-Halogenalkoxy, besonders C₁-C₂-Halogenalkyloxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
C₂-C₆-Alkenyloxy wie Ethenyloxy, 1-Propenyloxy, 2-Propenyloxy, 1-Methylethenyloxy, 1-Butenyloxy, 2-Butenyloxy, 3-Butenyloxy, 1-Methyl-1-propenyloxy, 2-Methyl-1-propenyloxy, 1-Methyl-2-propenyloxy, 2-Methyl-2-propenyloxy, 1-Pentenyloxy, 2-Pentenyloxy, 3-Pentenyloxy, 4-Pentenyloxy, 1-Methyl-1-butenyloxy, 2-Methyl-1-butenyloxy, 3-Methyl-1-butenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-1-propenyloxy, 1,2-Dimethyl-2-propenyloxy, 1-Ethyl-1-propenyloxy, 1-Ethyl-2-propenyloxy, 1-Hexenyloxy, 2-Hexenyloxy, 3-Hexenyloxy, 4-Hexenyloxy, 5-Hexenyloxy, 1-Methyl-1-pentenyloxy, 2-Methyl-1-pentenyloxy, 3-Methyl-1-pentenyloxy, 4-Methyl-1-pentenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy;
Cyano; Cyanato, Thiocyanato; Nitro; Amino; Hydroxy; Carboxyl;
C₁-C₆-Alkylamino wie Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino und 1-Ethyl-2-methylpropylamino;
Di-C₁-C₆-alkylamino, besonders Di-C₁-C₄-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Di-methylethyl)-N-(1-methylethyl)amino, N-Butyl-N-(1-methyl-propyl)amino, N-Butyl-N-(2-methylpropyl)amino, N-Butyl-N-(1,1-dimethylethyl)amino, N-(1-Methylpropyl)-N-(2-methyl-propyl)amino, N-(1,1-Dimethylethyl)-N-(1-methylpropyl)amino und N-(1,1-Dimethylethyl)-N-(2-methylpropyl)amino;
Phenyl, Phenoxy, Phenylthio und Phenylamino,
welche ihrerseits ein bis fünf Halogenatome wie vorstehend genannt;
und/oder eine bis fünf C₁-C₆-Alkoxygruppen wie vorstehend genannt;
und/oder eine bis fünf C₁-C₆-Alkylthiogruppen wie vorstehend genannt;
und/oder eine bis fünf C₁-C₄-Halogenalkylgruppen wie vorstehend genannt;
und/oder eine bis vier C₁-C₆-Alkoxyiminomethylgruppen wie Methoxyiminomethyl, Ethoxyiminomethyl, n-Propoxyiminomethyl, n-Butoxyiminomethyl, n-Pentoxyiminomethyl, n-Hexoxyiminomethyl, Allyloxyiminomethyl, Benzyloxyiminomethyl, iso-Propoxyiminomethyl, iso-Butoxyiminomethyl und tert.-Butoxyiminomethyl,
und/oder eine bis vier C₁-C₆-Alkoxyiminoethylgruppen wie Methoxyiminoethyl, Ethoxyimino-1-ethyl, n-Propoxyimino-1-ethyl, n-Butoxyimino-1-ethyl, n-Pentoxyimino-1-ethyl, n-Hexoxyimino-1-ethyl, Allyloxyimino-1-ethyl, Benzyloxyimino-1-ethyl; und/oder Phenyl, Phenoxy und Benzyloxy;
und/oder eine C₁-C₄-Alkyliminomethylgruppe wie Methyliminomethyl und Ethyliminomethyl;
und/oder eine bis vier C₃-C₆-Cycloalkylgruppen wie Cyclopropyl, Cyclobutyl, Cyclopentyl und Cyclohexyl, tragen können.

Die neuen Verbindungen der allgemeinen Formel I können bei der Herstellung aufgrund der C=C bzw. C=N-Doppelbindungen als E/Z-Isomerengemische anfallen. Diese können in üblicher Weise durch Kristallisation oder Chromatographie in die einzelnen Komponenten aufgetrennt werden.

Sowohl die einzelnen Isomeren Verbindungen als auch ihre Gemische werden von der Erfindung umfaßt und sind als Fungizide brauchbar.

Die Herstellung der Verbindungen der allgemeinen Formel I gemäß Anspruch 1 erfolgt beispielsweise wie in Schema 1 beschrieben (Z¹, Z² gleich H). 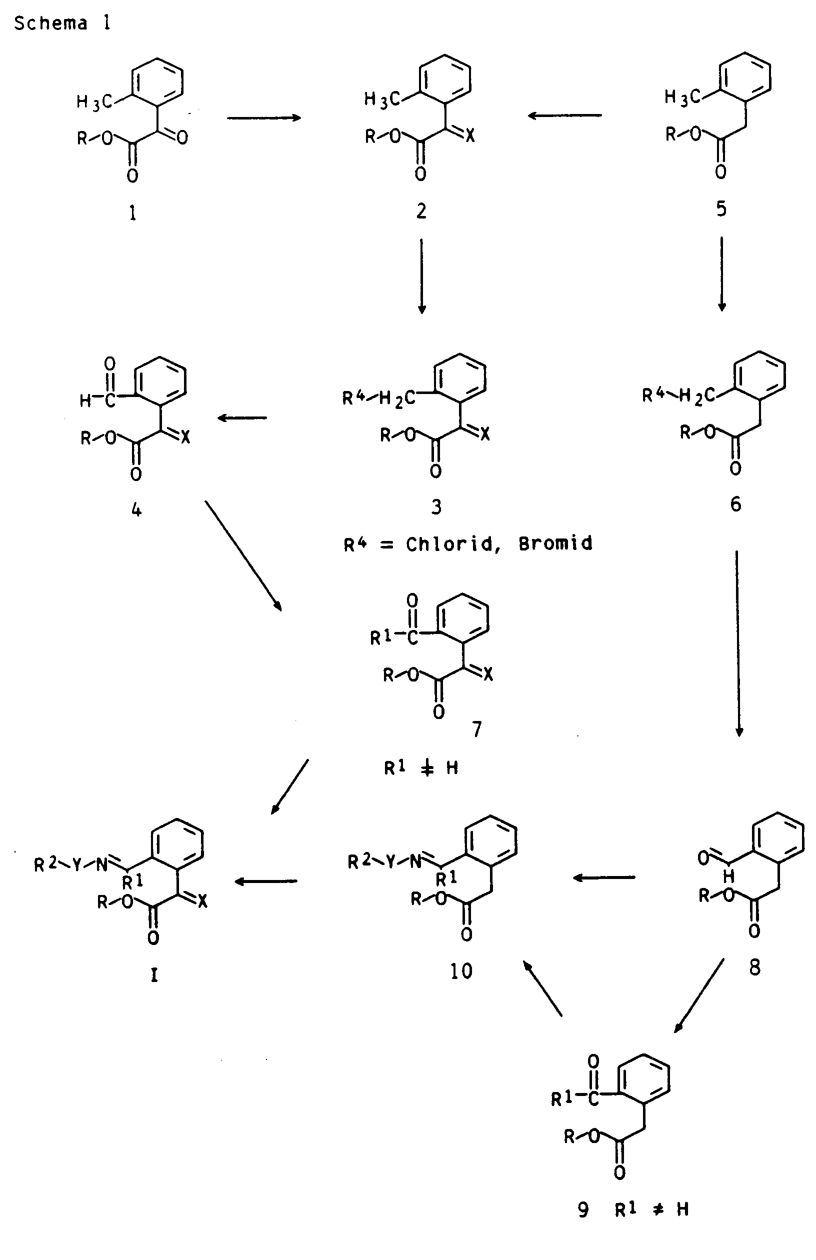
Die Verbindungen der Formel I mit X = N-O-Alkyl lassen sich aus 1 a) durch Umsetzung mit O-Alkylhydroxylamin-hydrochlorid oder b) mit Hydroxylamin-hydrochlorid und nachfolgender Alkylierung mit einem Alkylierungsmittel (wie z.B. Alkyljodid, Dialkylsulfat etc.) herstellen (vgl. DE 36 23 921)

Ebenso kann analog zur Methode in EP 254 426 ein Phenylessigester der Formel 8 oder 9 mit einer Base (wie z.B. NaOMe, NaH, K-tert.-Butylat etc.) in einem Lösungsmittel (wie z.B. Diethylether, Toluol, tert.-Butanol etc.) in sein Anion überführt und mit einem geeigneten Nitrosierungsmittel (wie z.B. Methylnitrit, Amylnitrit, tert.-Butylnitrit etc.) oximiert werden. Das resultierende Oxim wird mit einem Alkylierungsmittel (wie z.B. Alkyljodid, Dialkylsulfat) alkyliert.

Die Zwischenprodukte der Formeln 3 und 6 lassen sich aus den Verbindungen 2 und 5 herstellen, indem man diese nach bekannten Methoden halogeniert, z.B. mit Chlor, Brom, N-Bromsuccinimid in einem inerten Lösungsmittel (z.B. CCl₄, Cyclohexan) unter Belichtung mit z.B. einer Hg-Dampflampe oder mit Radikalstartern wie z.B. Dibenzoylperoxid.

Benzaldehyde der Formeln 4 und 8 sind entweder bekannt oder können durch Oxidation der Benzylhalogenide 3 oder 6 z.B. mit N-Methylmorpholin-N-Oxid in einem inerten Lösungsmittel wie z.B. CCl₄ oder Cyclohexan hergestellt werden. Außerdem kann man die Benzaldehyde der Formeln 4 und 8 herstellen, wenn man die entsprechenden Benzylhalogenide mit einem Oxidationsmittel wie z.B. Silbernitrat in Methanol oder Ethylenglycolmonomethylether [vgl. J. Am. Chem. Soc. 78, 1689 (1956)] behandelt.

Die Herstellung der Verbindungen der allgemeinen Formeln 7 und 9 kann beispielsweise wie in Schema 2 beschrieben erfolgen. 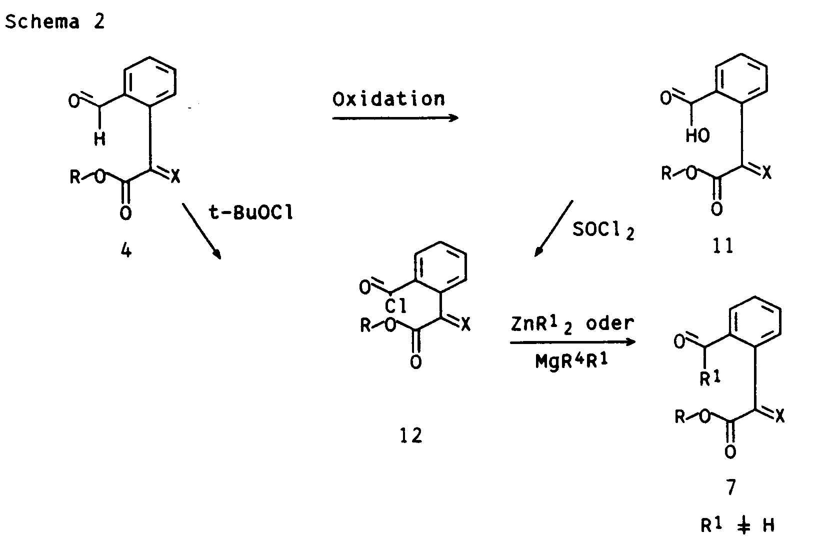
Die Benzaldehyde 4 und 8 können in bekannter Weise in die Carbonsäuren 11 und 13 umgewandelt werden [vgl. Organikum 15. Auflage, 447B (1977)].

Aus den so erhaltenen Carbonsäuren 11 und 13 lassen sich in an sich bekannter Weise die Säurechloride 12 und 14 herstellen [vgl. Organikum 15. Auflage, 526 fB (1977)].

Alternativ können die Säurechloride 12 und 14 auch direkt aus den Benzaldehyden 4 und 8 erhalten werden, wenn man sie beispielsweise mit t-BuOCl in einem inerten Lösungsmittel wie z.B. CCl₄ behandelt [vgl. D. Ginsburg, J. Amer. Chem. Soc. 73, 702 (1951)]. Die Ketone 7 und 9 erhält man beispielsweise aus den Säurechloriden 12 und 14 durch Umsetzung mit zinkorganischen Verbindungen vom Typ ZnR¹₂ analog (Org. React. 1954, 8, 28).

Die Darstellung der neuen Verbindungen der allgemeinen Formel I kann so erfolgen, daß man die neuen Carbonylverbindungen der Formel 7 mit einem substituierten Hydroxylamin der allgemeinen Formel 15 oder Hydrazin der allgemeinen Formel 16 oder mit einem Säureadditionssalz (z.B. Hydrochlorid, Hydrobromid) von 15 oder 16 umsetzt. 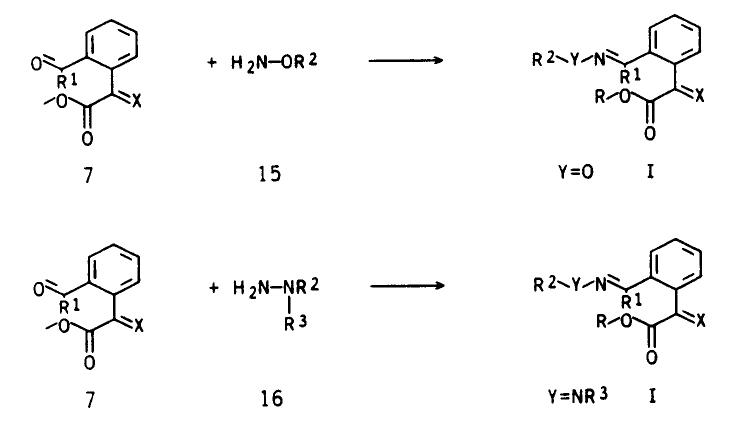
Die Reaktion kann in einem inerten Lösungs- oder Verdünnungsmittel (z.B. Methanol, Ethanol, Toluol) oder in einem Zweiphasensystem (z.B. Toluol/Wasser) durchgeführt werden. Außerdem kann es von Vorteil sein, der Reaktionsmischung eine Base (z.B. Triethylamin, Natriumcarbonat, Kaliumcarbonat, Natriumhydrogencarbonat, Kaliumhydrogencarbonat, Natriumhydroxid, Kaliumhydroxid) zuzusetzen.

Alternativ kann bei der Herstellung von I auch wie folgt vorgegangen werden (Schema 4, Z¹, Z² = H) 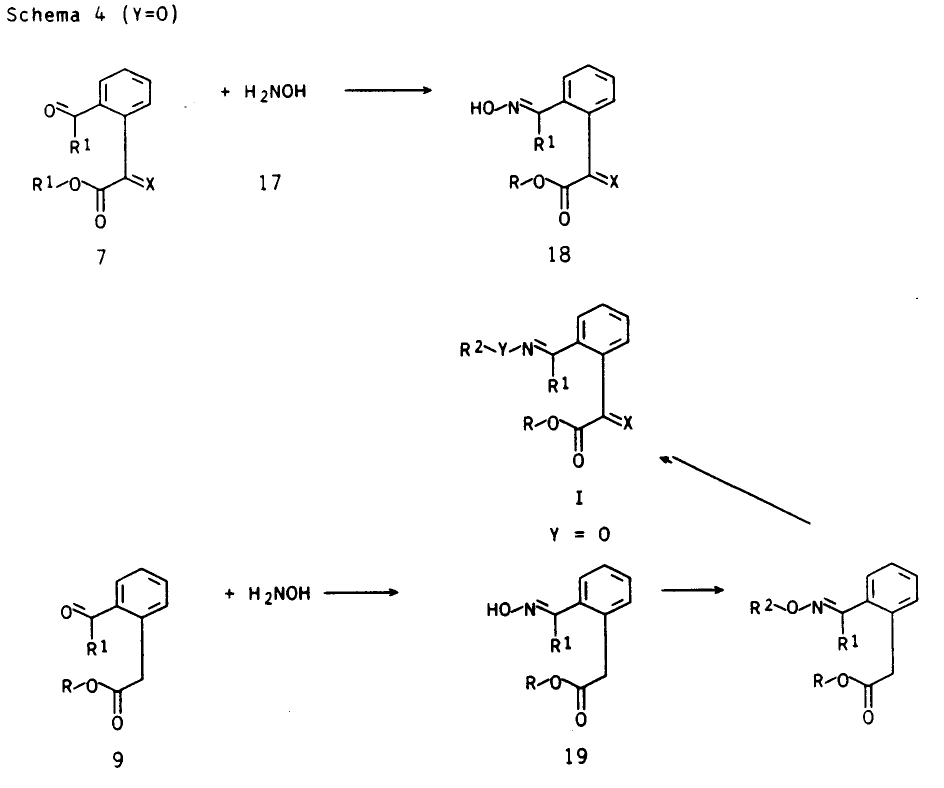
Die zur Herstellung der Verbindungen der allgemeinen Formel I (Y = 0) benötigten Oxime 18 und 19 (vgl. Schema 4) können aus den entsprechenden Carbonylverbindungen 7 und 9 in bekannter Weise (vgl. Houben-Weyl, Bd. 10/4 (1968)) durch Umsetzung mit dem Hydroxylamin 17 hergestellt werden.

Die Reaktion kann in einem inerten Lösungs- oder Verdünnungsmittel (z.B. Methanol, Ethanol, Toluol) oder in einem Zweiphasensystem (z.B. Toluol/Wasser) durchgeführt werden. Außerdem kann es von Vorteil sein, der Reaktionsmischung eine Base (z.B. Triethylamin, Natriumcarbonat, Kaliumcarbonat, Natriumhydrogencarbonat, Kaliumhydrogencarbonat, Natriumhydroxid, Kaliumhydroxid) zuzusetzen.

Die nachfolgende Alkylierung der Oxime 18 und 19 erfolgt in bekannter Weise mit einer Base (wie z.B. NaOMe, NaH, KOtBu in einem Lösungsmittel wie z.B. Diethylether, Toluol, Dimethylformamid etc.) (vgl. DE 3623921).

Hydroxylamine der allgemeinen Formel 15 (H₂N-OR²) sind entweder bekannt oder können nach bekannten Methoden analog Houben-Weyl X/1, 1192, 1183 hergestellt werden.

Hydrazinderivate der allgemeinen Formel 16 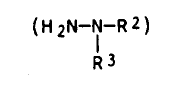
sind entweder bekannt oder können ebenso nach bekannten Methoden analog Houben-Weyl X/2, S. 271, 280, 740 hergestellt werden.

Ein weiterer Zugang zu den Verbindungen der allgemeinen Formel I wird in Schema 5 beschrieben (Z¹, Z² = H, R⁵ = Methyl, Ethyl). 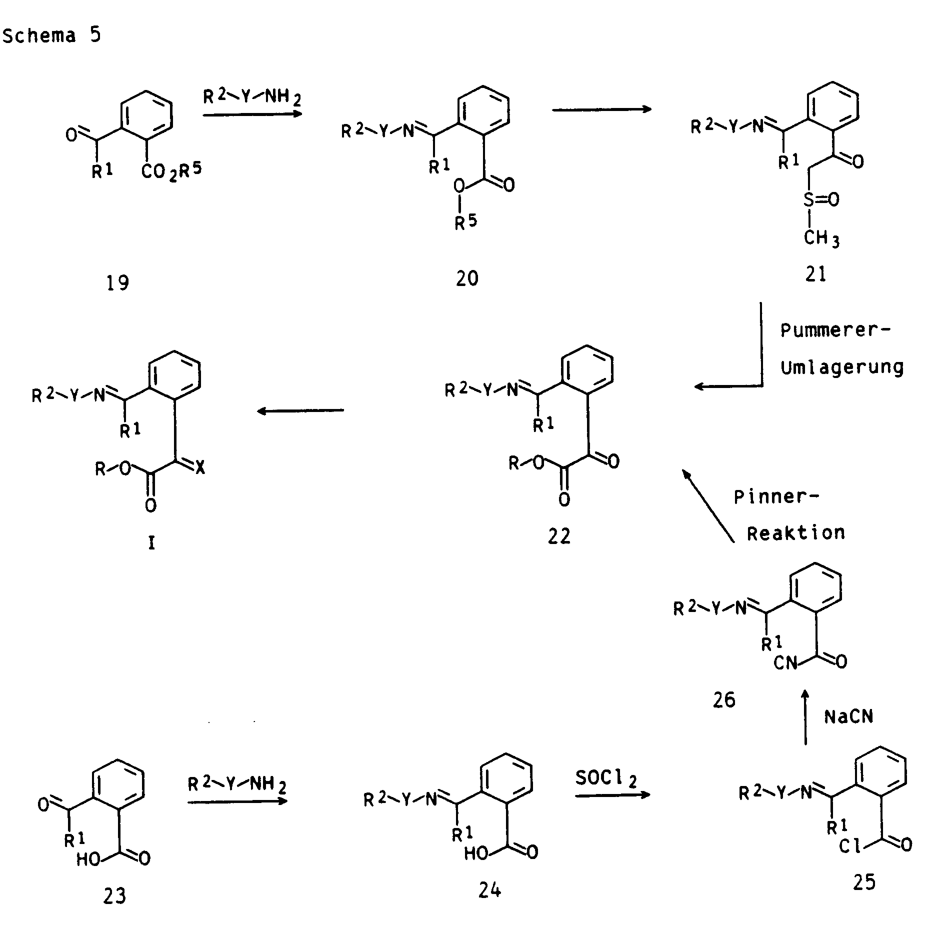
Ausgehend von den Carbonsäureestern der Formel 20 kann man durch Anlagern von Dimethylsulfoxid in Anwesenheit einer starken Base (wie z.B. NaOCH₃, KOtBu) die β-Ketosulfoxide 21 erhalten [vgl. J. Am. Chem. Soc. 88, 5498 (1966)], die nach Bromierung und anschließendem Verkochen mit einem Alkohol (wie z.B. Methanol, Ethanol) in Gegenwart einer Säure in einer sogenannten "Pummerer-Umlagerung" die α-Ketoester 22 ergeben [Synthesis, 47 (1982)].

Alternativ kann man die α-Ketoester der Formel 22 durch Anwendung der sogenannten "Pinner-Reaktion" bei den Acylcyaniden der Formel 26 erhalten [vgl. S. Hünig et al., Angew. Chemie 94, 1 (1982)].

Die Überführung der Carbonsäuren 24 in die Acylcyanide 26 erfolgt in üblicher, an sich bekannter Weise [vgl. "Organikum": 16. Auflage, Berlin 1986, S. 423f und J.M. Photis, Tetr. Lett. 21, 3539-3540 (1980)].

Die Zwischenprodukte der allgemeinen Formel 19 kann man ausgehend von O-Hydroxyphenylketonen der Formel 27 analog Tetr. Letters, 6781 (1990) (Schema 6) herstellen. 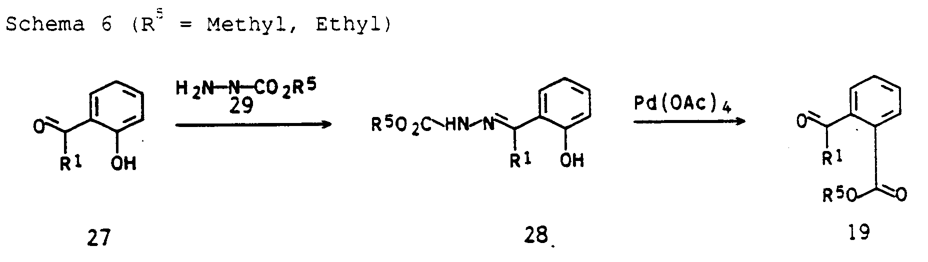
Die folgenden Vorschriften und Beispiele sollen die Herstellung der neuen Wirkstoffe und der neuen Zwischenprodukte erläutern.

### Vorschrift 2

### Herstellung von 2-Formylphenyl-glyoxylsäuremethylester-O-methyloxim

20 g (0,070 mol) 2-(Brommethyl)-phenylglyoxylsäuremethylester-O-methyloxim, 32 g (0,237 mol) N-Methylmorpholin-N-oxid-monohydrat und 300 ml CCl₄ werden zusammengegeben und zum Rückfluß erhitzt. Nach 9 Stunden Rückfluß wird abfiltriert, die organische Phase mit Wasser sowie mit 2 N Salzsäure gewaschen, getrocknet und eingeengt. Der Rückstand wird aus Pentan umkristallisiert. Man erhält 9,0 g Produkt (58 %) als farblose Kristalle.
¹H-NMR (CDCl₃): 3,9(s,3H); 4,0(s,3H); 7,39-8,0(m,4H); 9,98 ppm (s,1H).

### Vorschrift 3

### Herstellung von 1-Brom-2-methoxymethyl-benzol

685 g (2,74 mol) o-Brombenzylbromid werden zusammen mit 2,74 mol einer 30 %igen Natriummethylat-Lösung in Methanol 15 Stunden unter Rückfluß erhitzt. Nach dem Abkühlen wird eingeengt, in Essigester aufgenommen und mit Wasser gewaschen. Nach dem Trocknen der organischen Phase über Natriumsulfat wird eingeengt. Man erhält 478,2 g (87 %) Produkt als farblose Flüssigkeit.
¹H-NMR (CDCl₃): 3,5(s,3H); 4,58(s,2H);7,08-7,6ppm (m,4H).

### Vorschrift 4

### Herstellung von 2-Methoxymethyl-phenylglyoxylsäuremethylester

Zu einer aus 12,0 g (0,46 mol) Magnesiumspänen und 99,5 g (0,5 mol) 1-Brom-2-methoxymethyl-benzol in 500 ml THF (Tetrahydrofuran) hergestellten Grignardlösung werden 500 ml etherische 1 N Zinkchloridlösung zugetropft. Anschließend wird 1 Stunde unter Rückfluß erhitzt und die Lösung von 61,2 g (0,5 mol) Oxalsäuremethylesterchlorid in 100 ml THF bei einer Innentemperatur von -10°C zugetropft. Man läßt auf Raumtemperatur (20°C) kommen, rührt noch 20 Stunden bei Raumtemperatur weiter und hydrolysiert mit Ammoniumchloridlösung. Nach dem Extrahieren mit Ether werden die vereinigten Etherphasen mit Wasser gewaschen, getrocknet und eingeengt. Man erhält 60 g Produkt (57,7 %) als eine leicht bewegliche Flüssigkeit.
¹H-NMR (CDCl₃): 3,38(s,3H); 3,98(s,3H);4,8(s,2H); 7,3-7,7 ppm (m,4H).

### Beispiel 1

### Herstellung von 2-(Methoxyimino-O-[(2-methyl)-phenylmethyl]-phenylglyoxylsäure-methylester-O-methyloxim

Die Lösung von 6 g (27 mmol) 2-Formylphenyl-glyoxylsäuremethylester-O-methyloxim in 150 ml Methanol wird mit 4 g (29 mmol) 2-Methyl-O-benzylhydroxylamin versetzt und 8 Stunden bei 65°C gerührt. Anschließend läßt man auf Raumtemperatur abkühlen, nimmt das Reaktionsgemisch in Essigester auf und wäscht die organische Phase mit Wasser. Nach dem Trocknen über Natriumsulfat wird das Lösungsmittel entfernt und man erhält 9 g (98%) des oben genannten Oximethers in Form gelblicher Kristalle (Verbindung Nr. 1.35, Tabelle I).
Fp.: 80-83°C
¹H-NMR (CDCl₃) : 2,4 (s,3H); 3,8 (s,3H); 3,98 (s,3H); 5,2 (s,2H); 7,05-7,8 (m,8H); 8,05 (s,1H) ppm.

In entsprechender Weise können die in den folgenden Tabellen aufgeführten Verbindungen hergestellt werden.

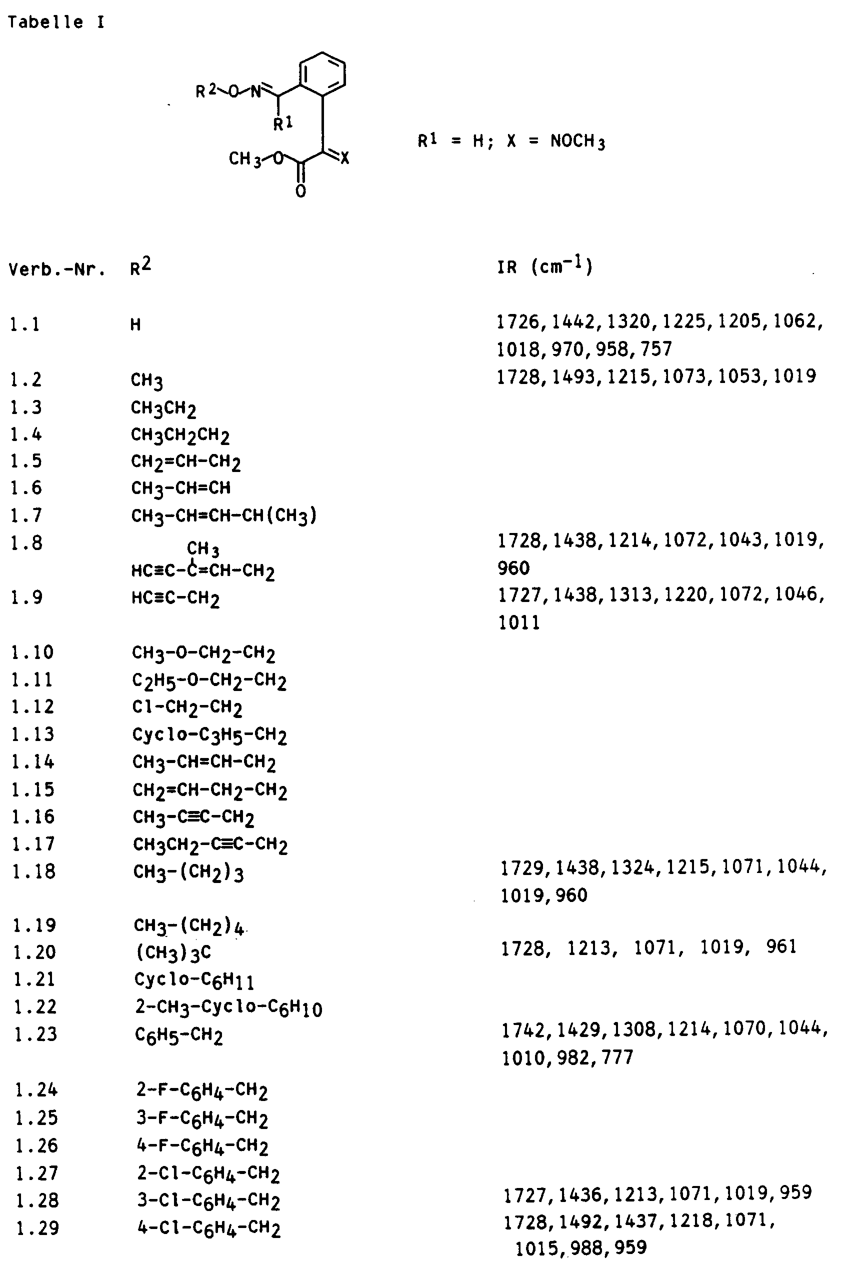

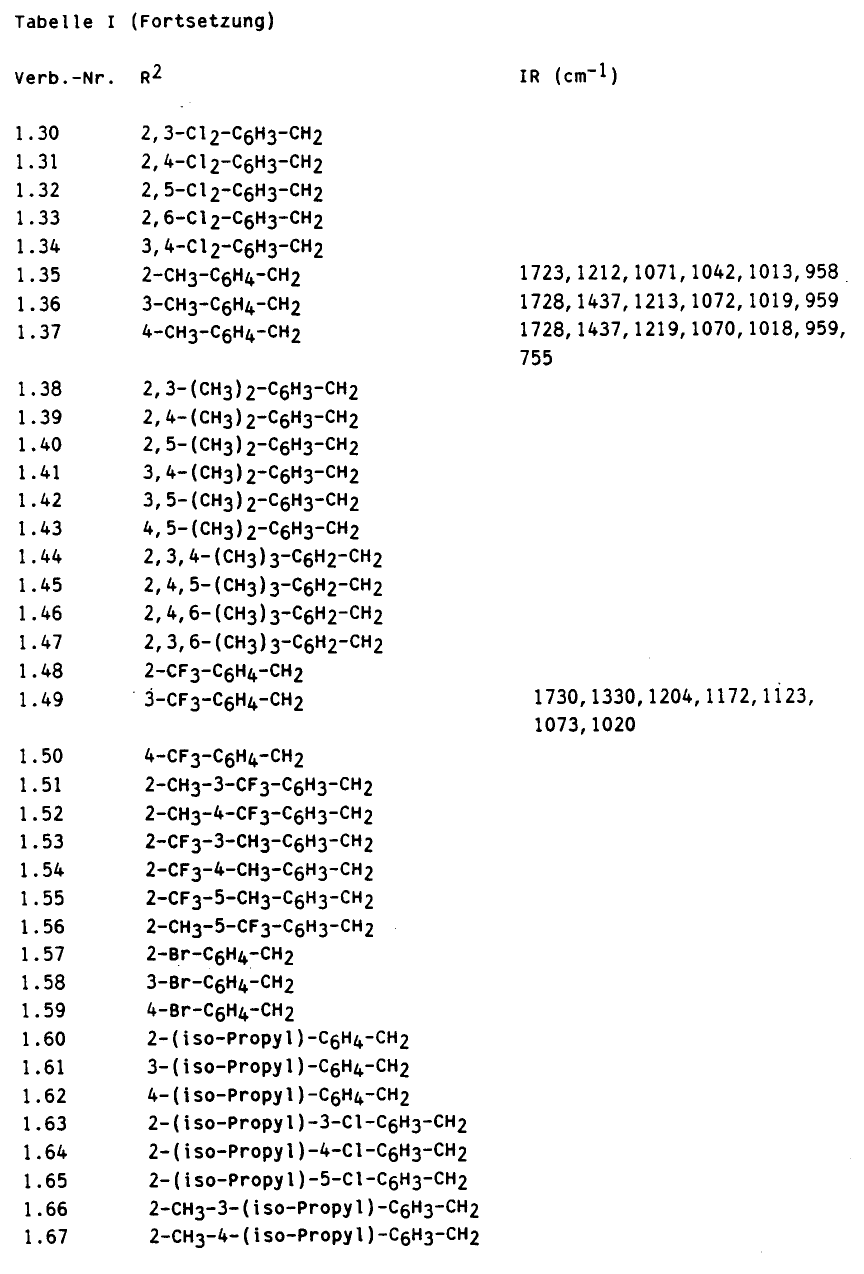

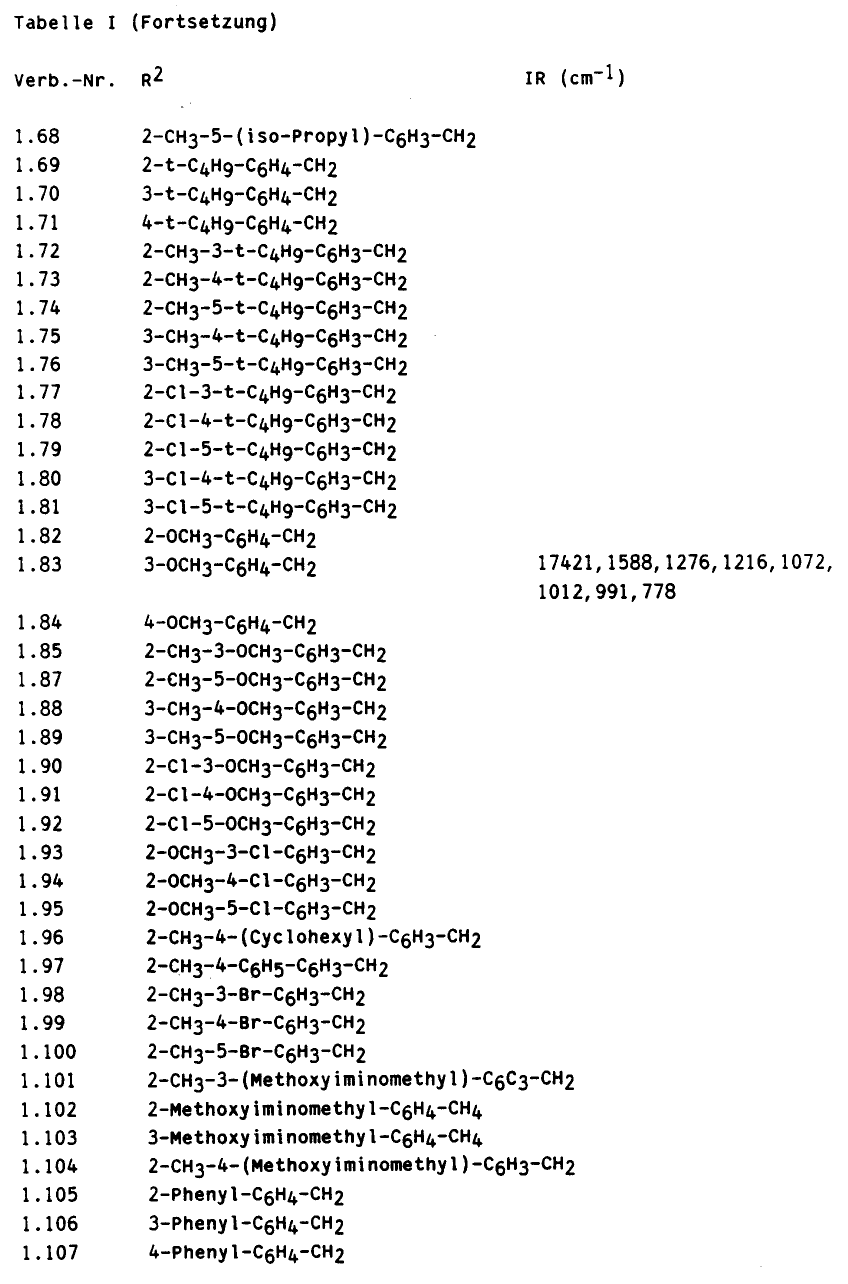

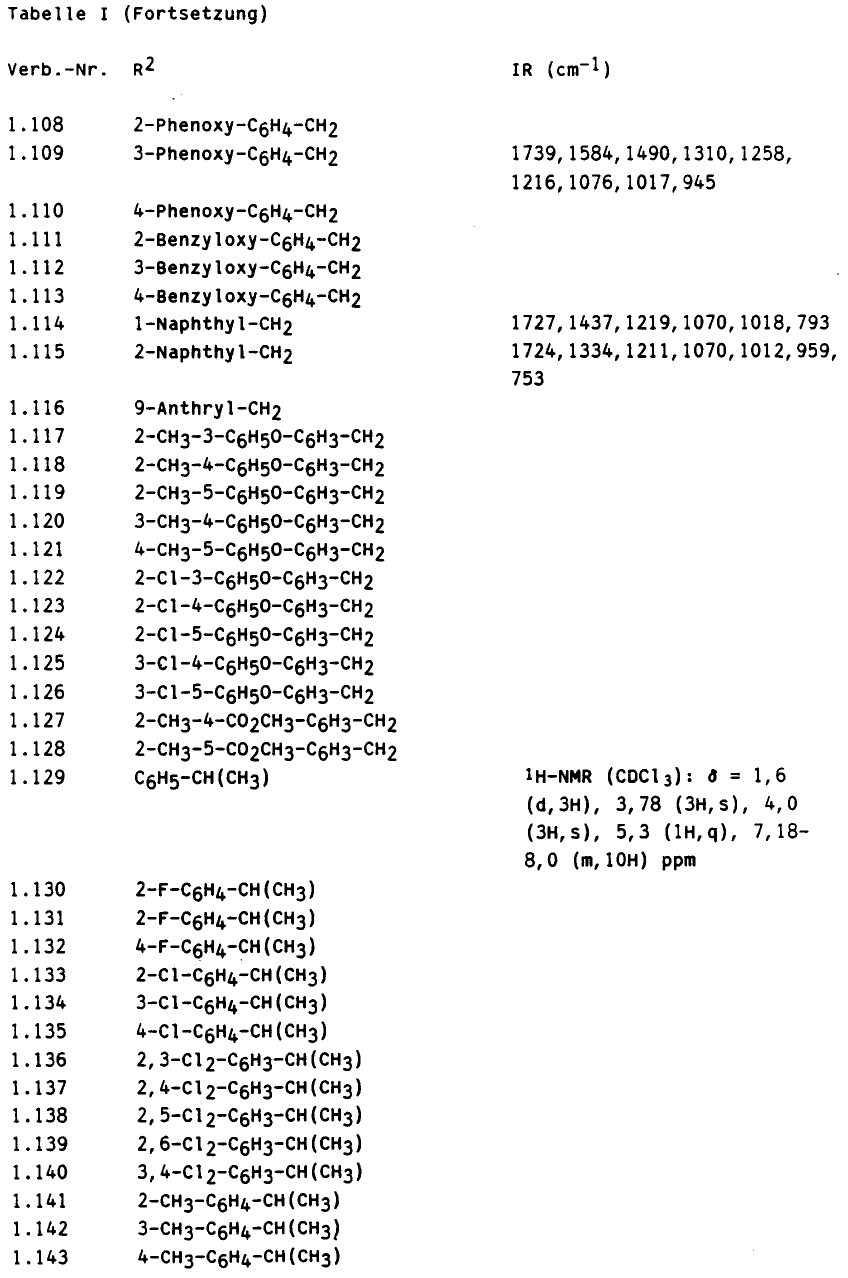

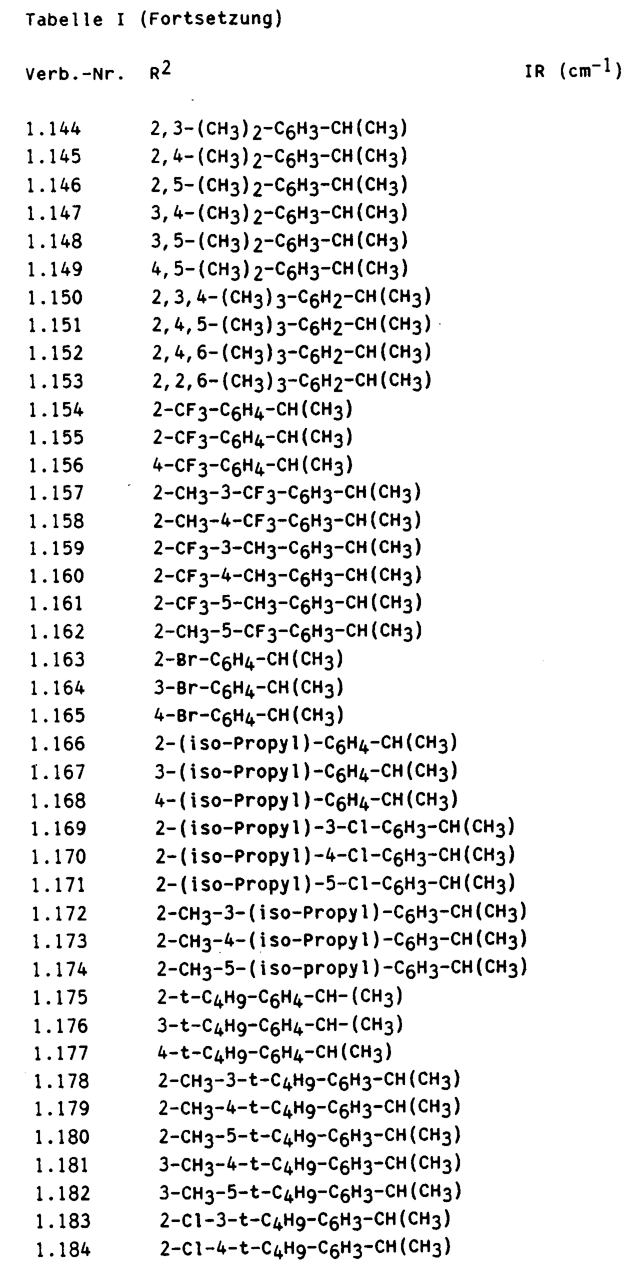

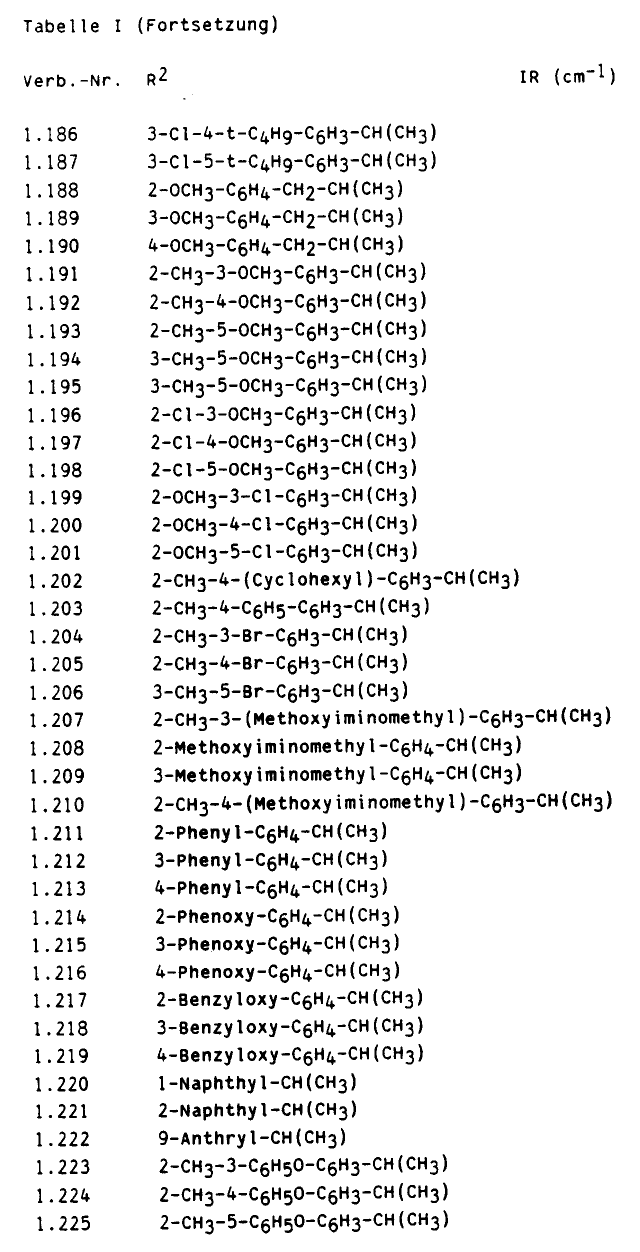

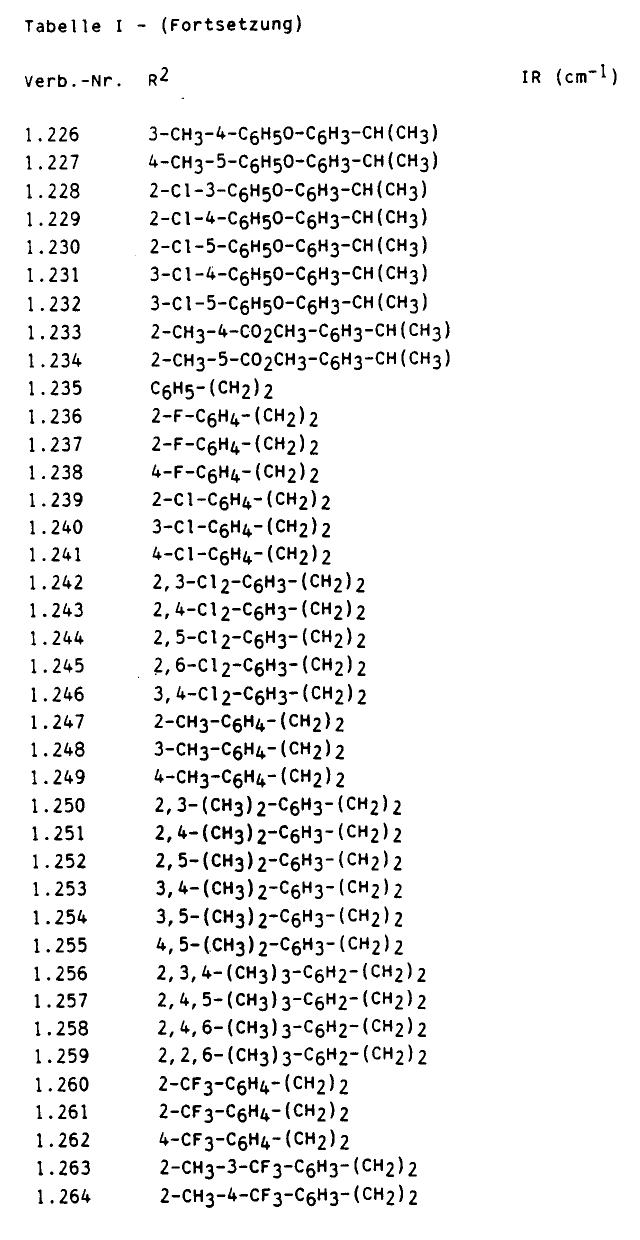

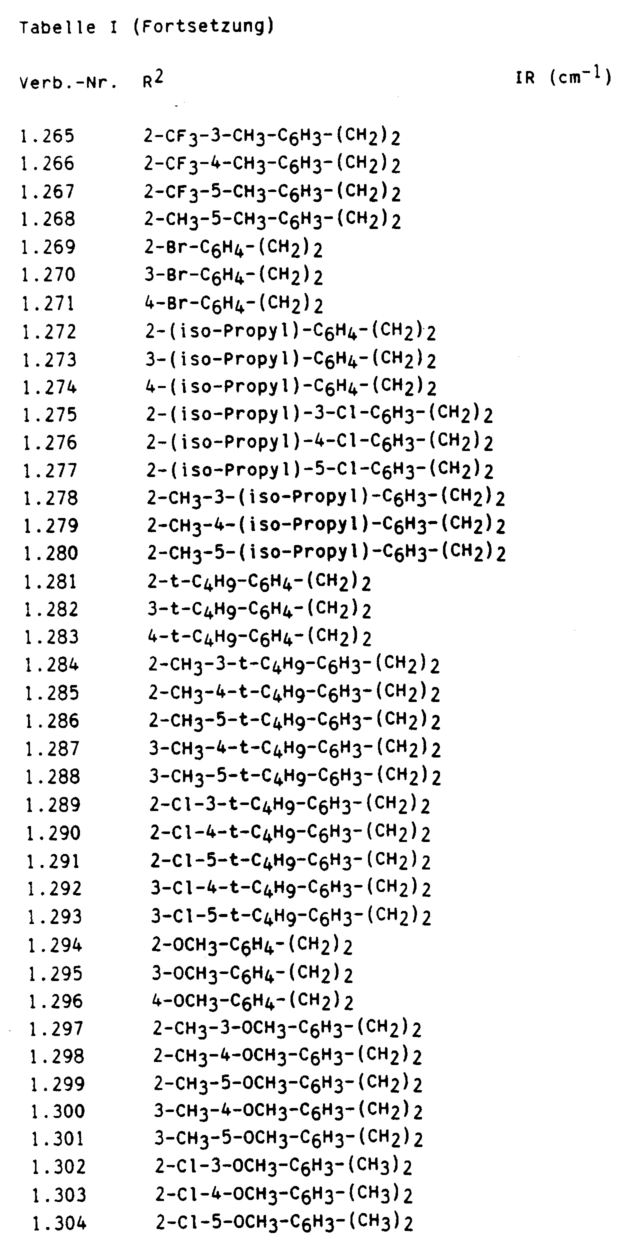

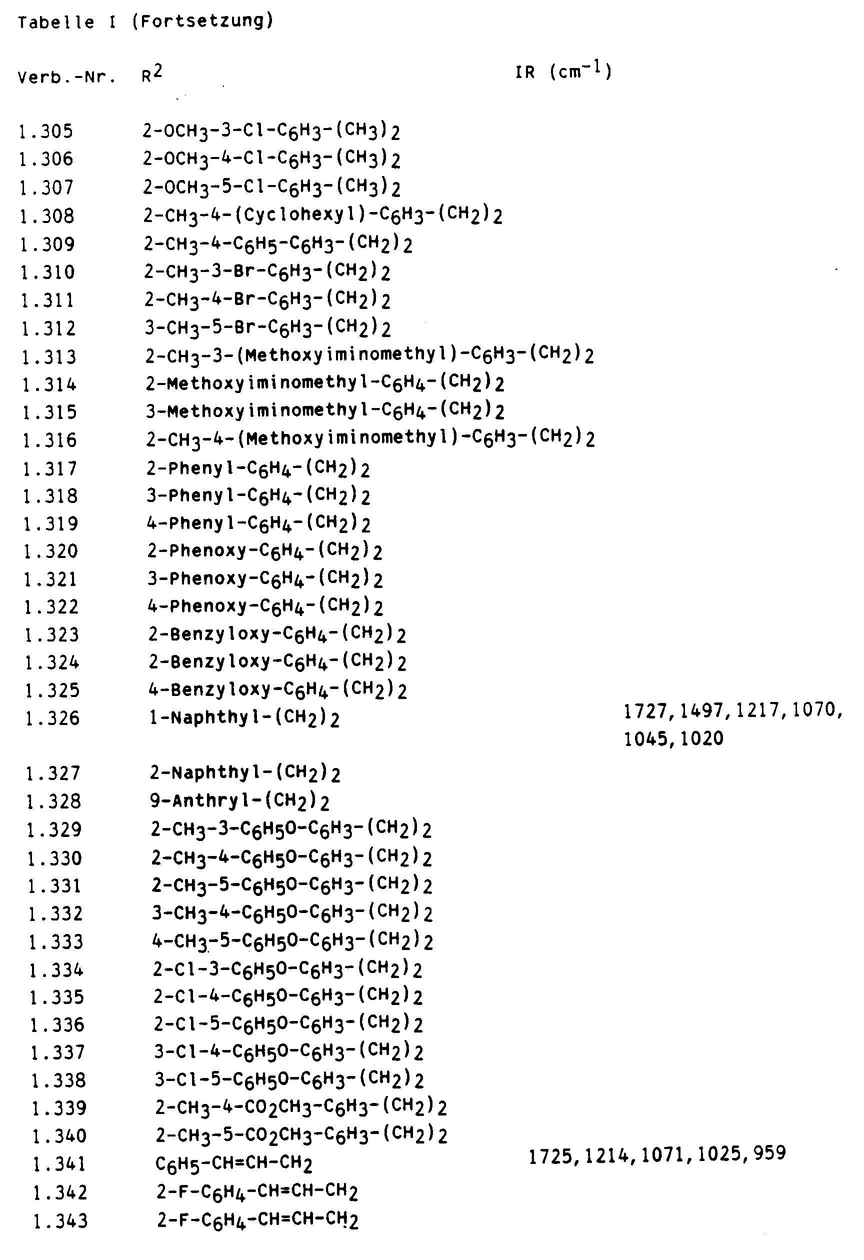

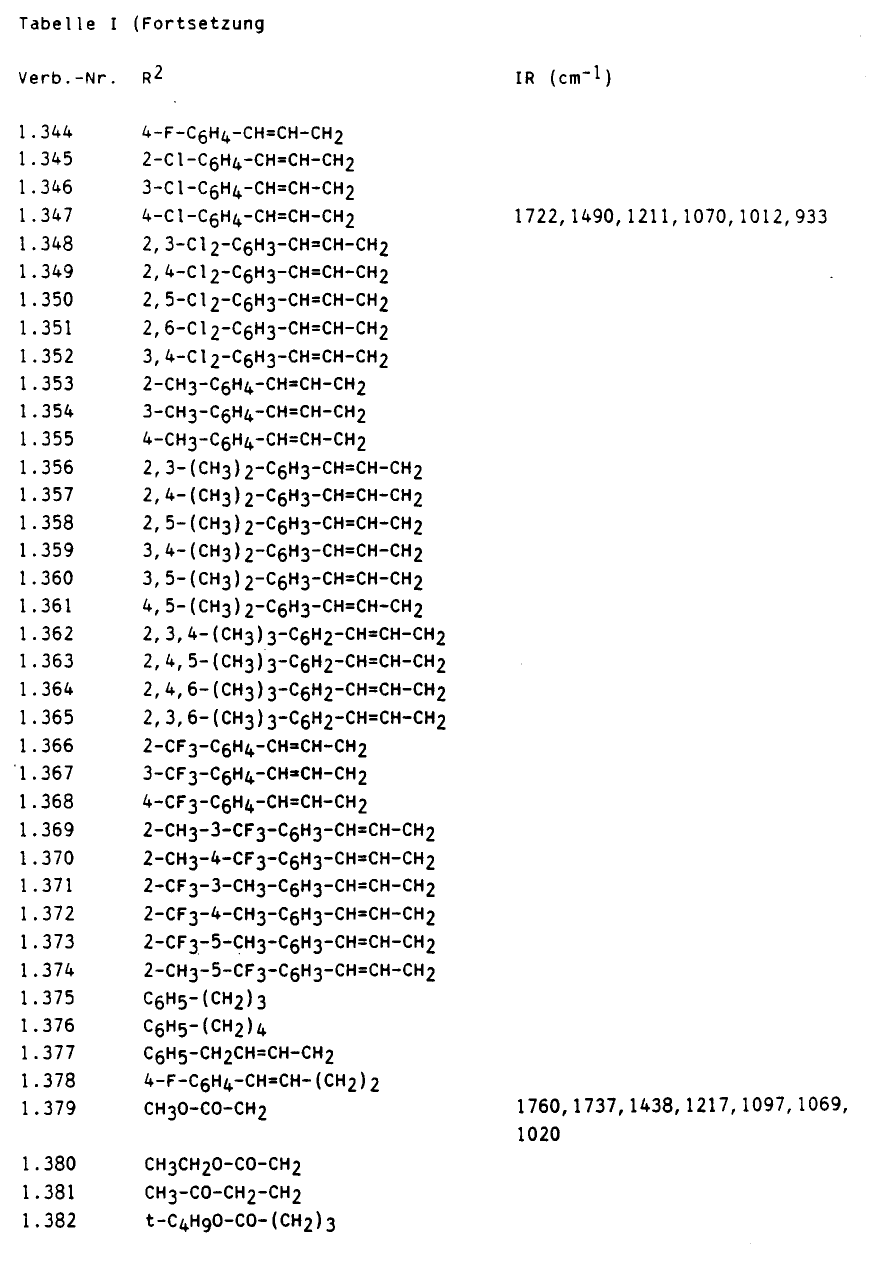

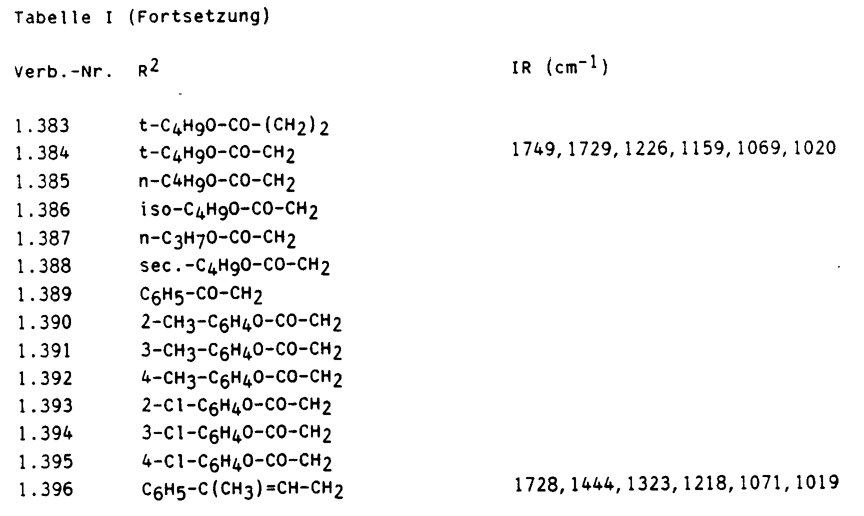

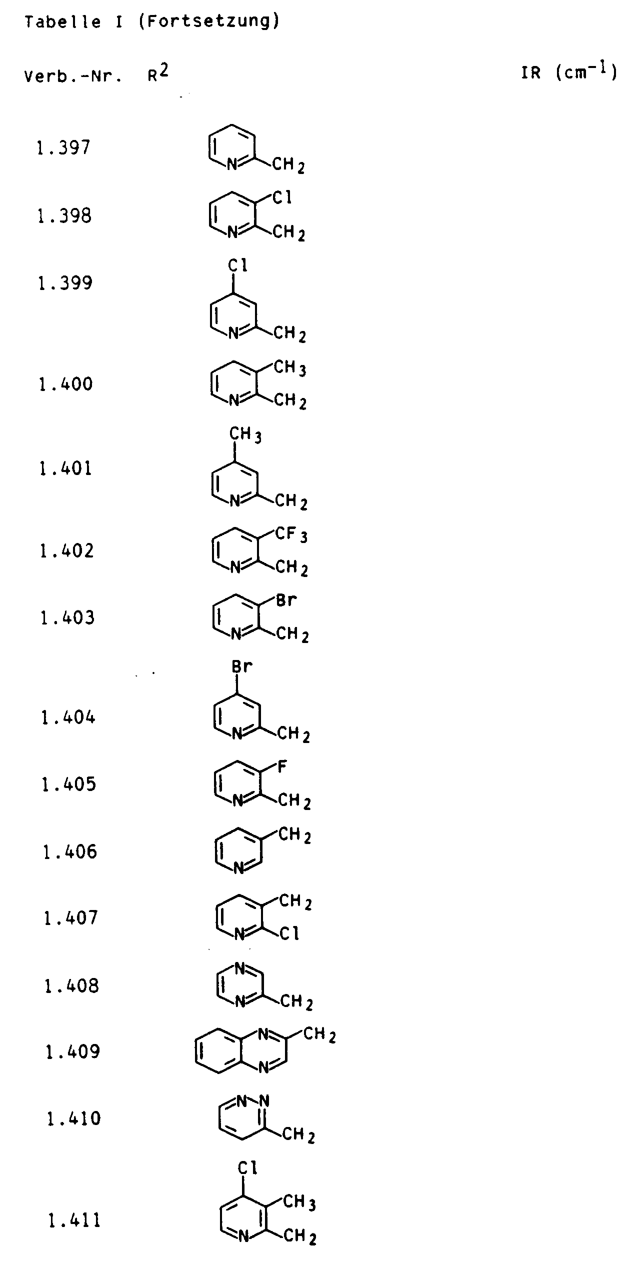

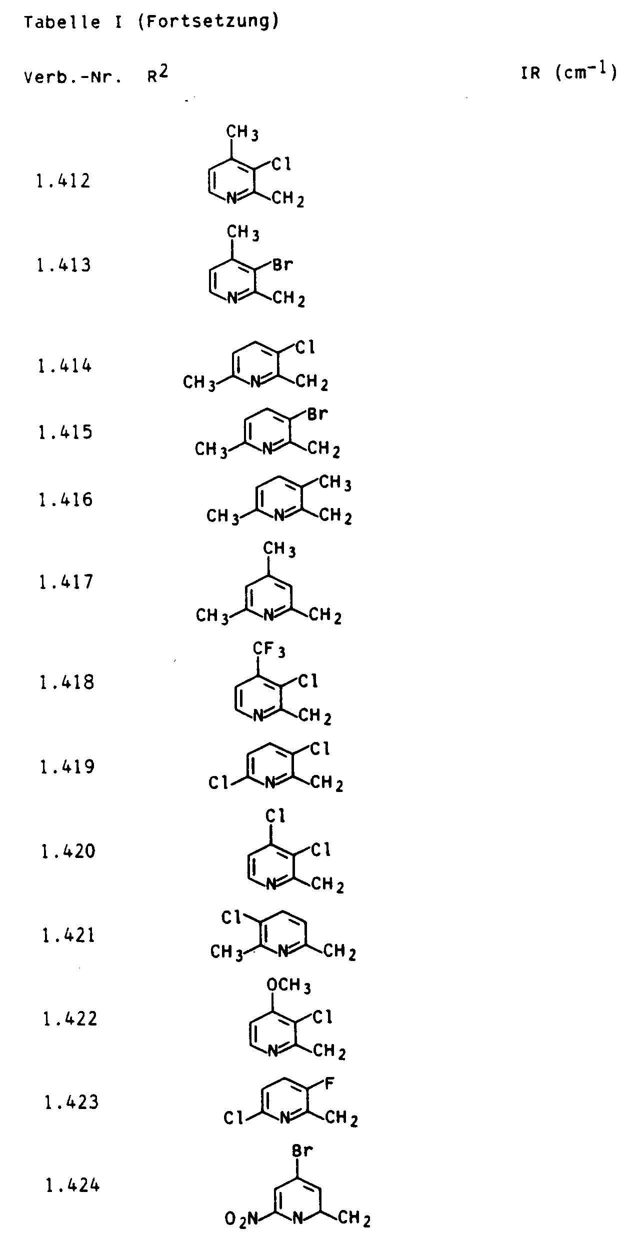

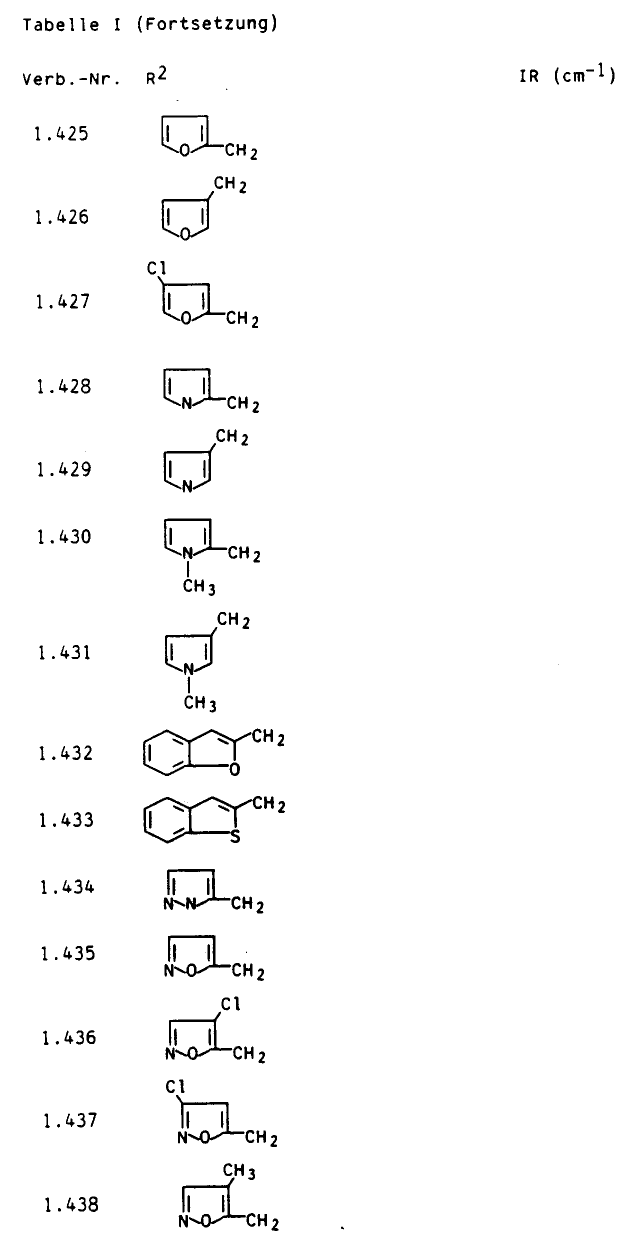

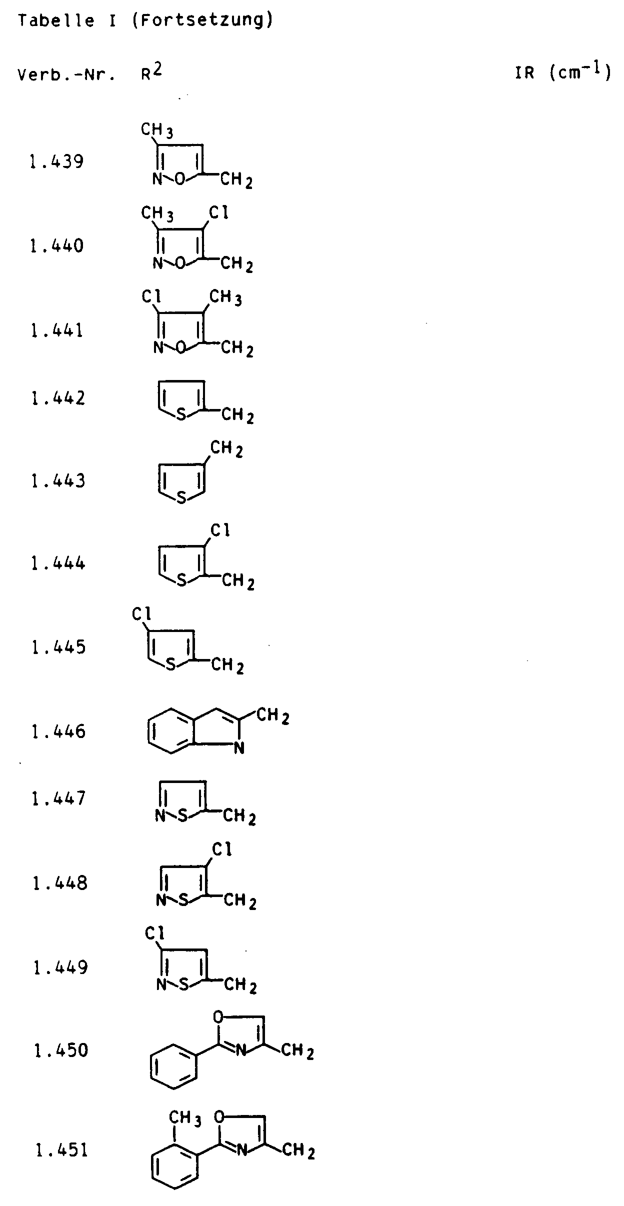

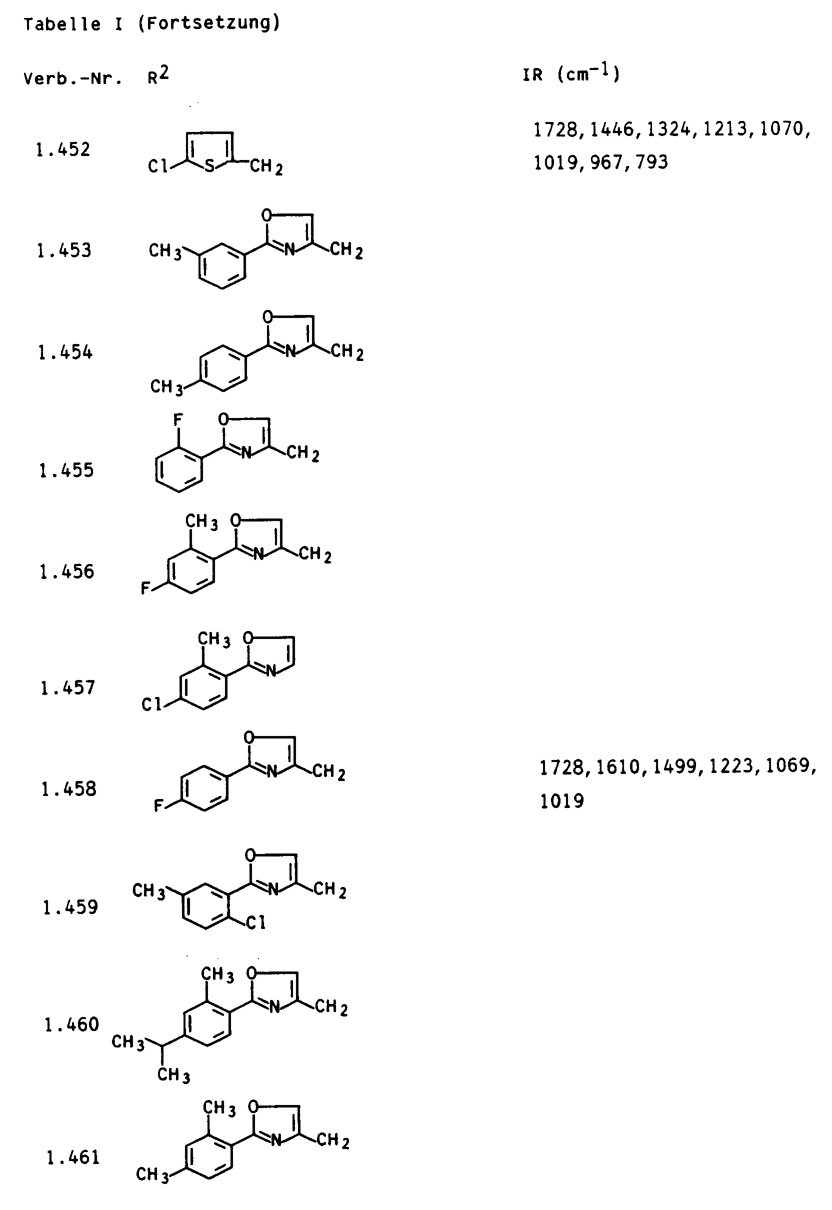

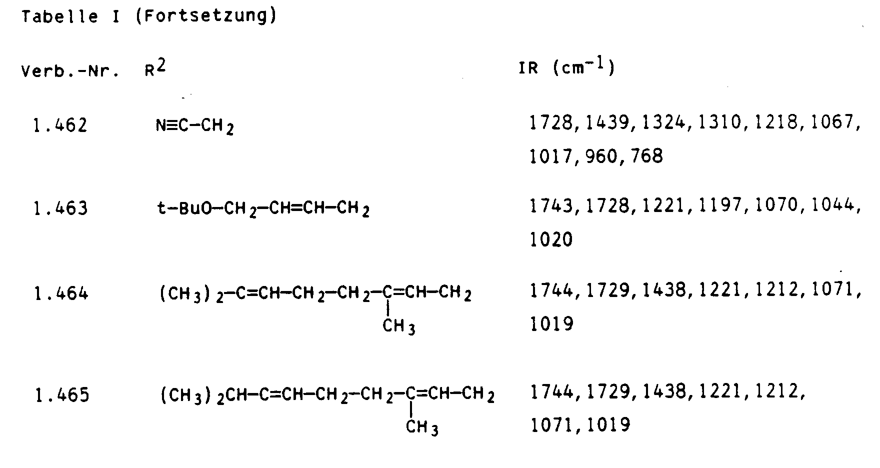

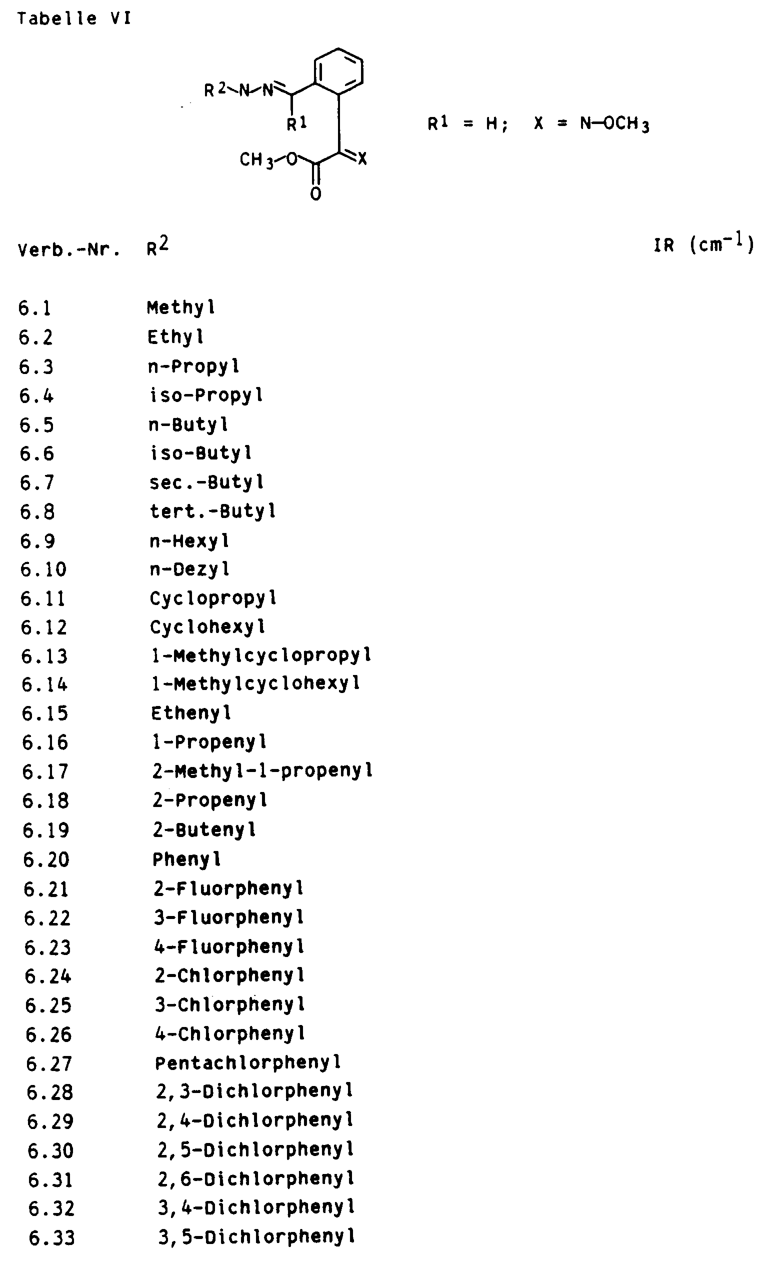

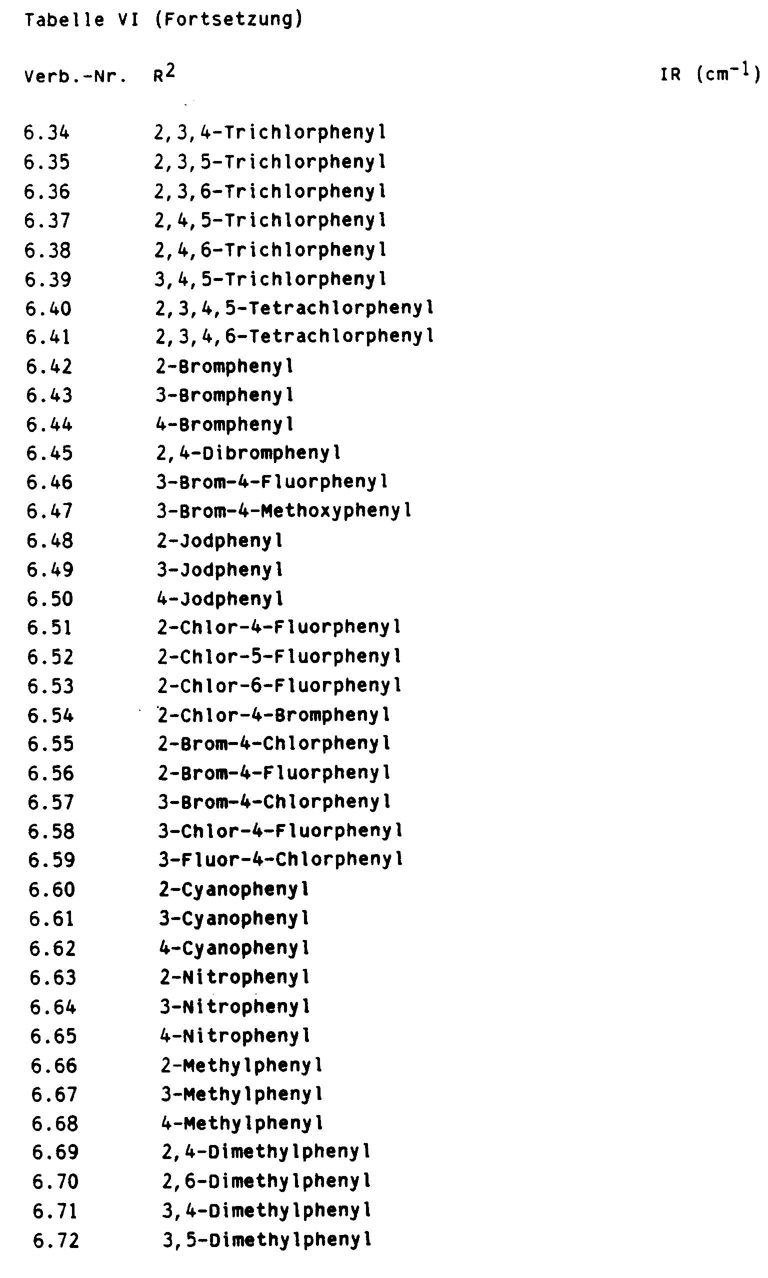

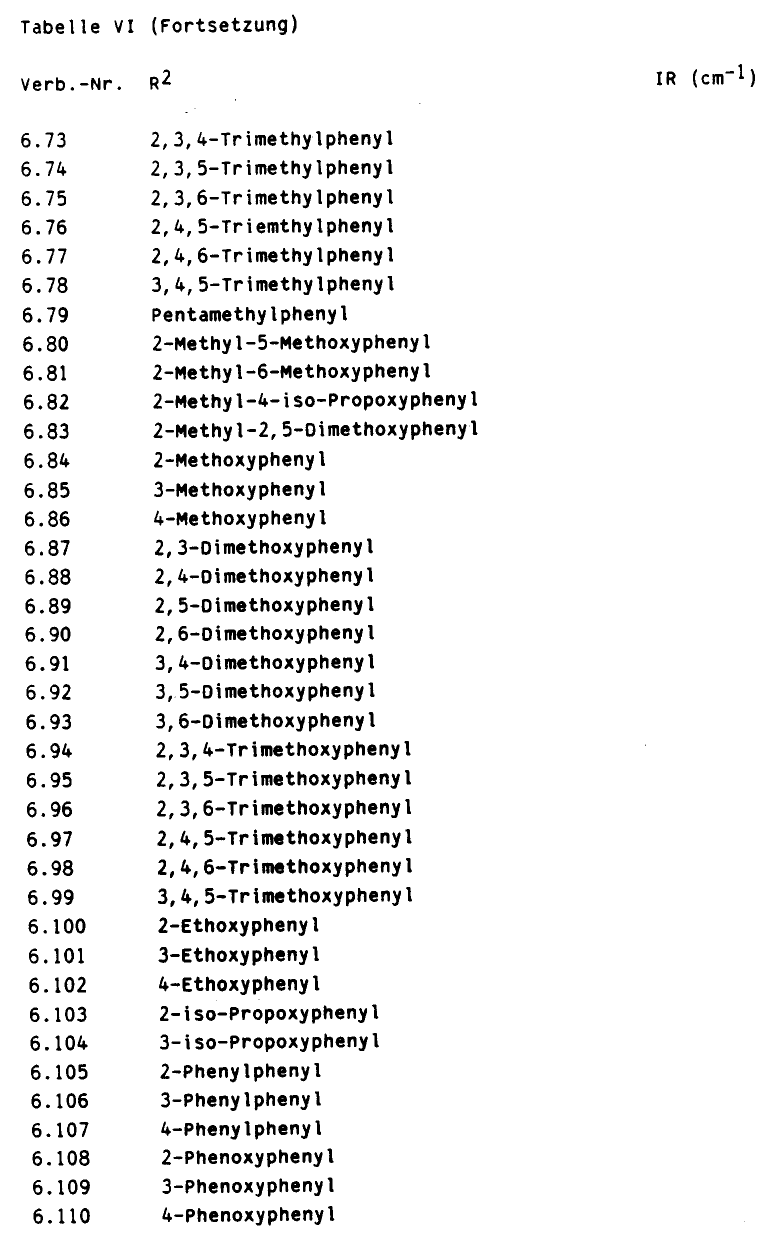

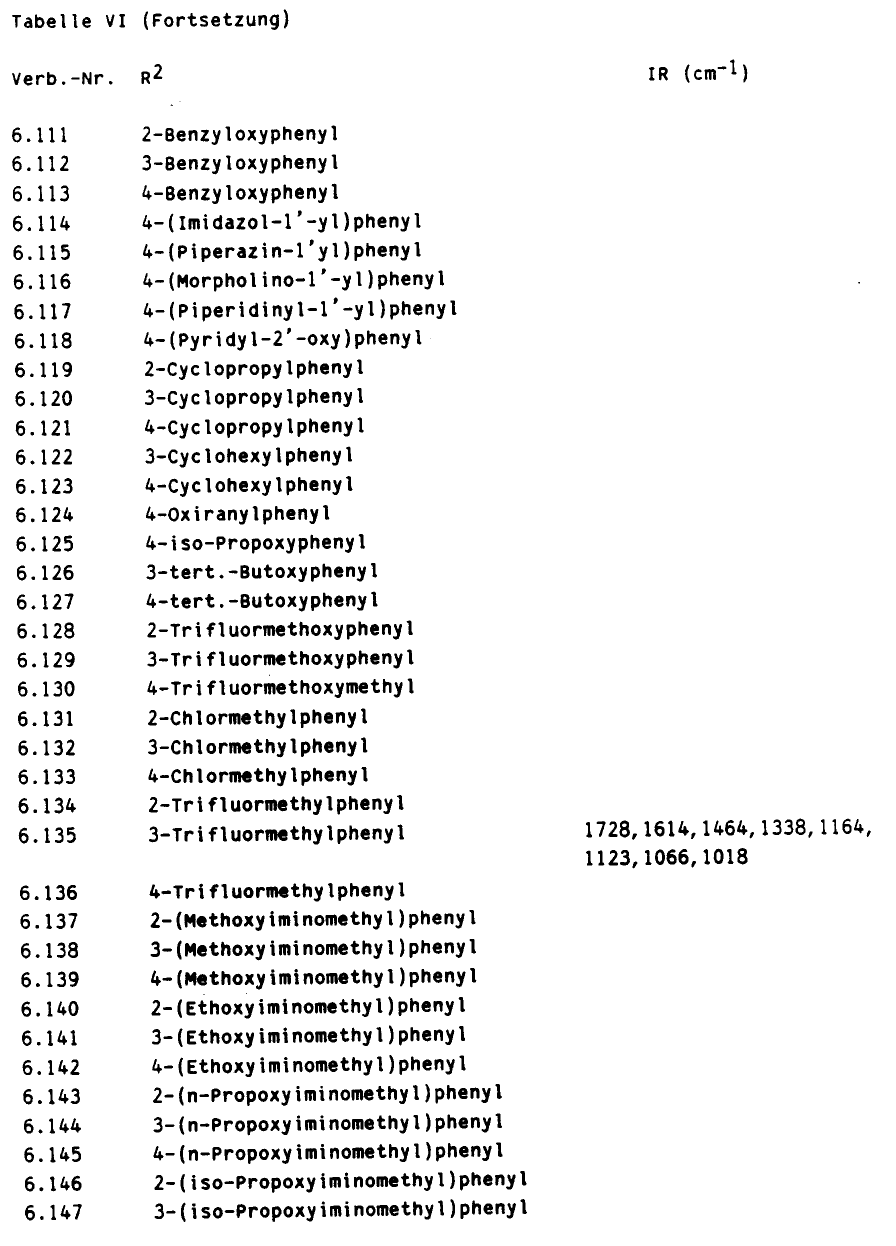

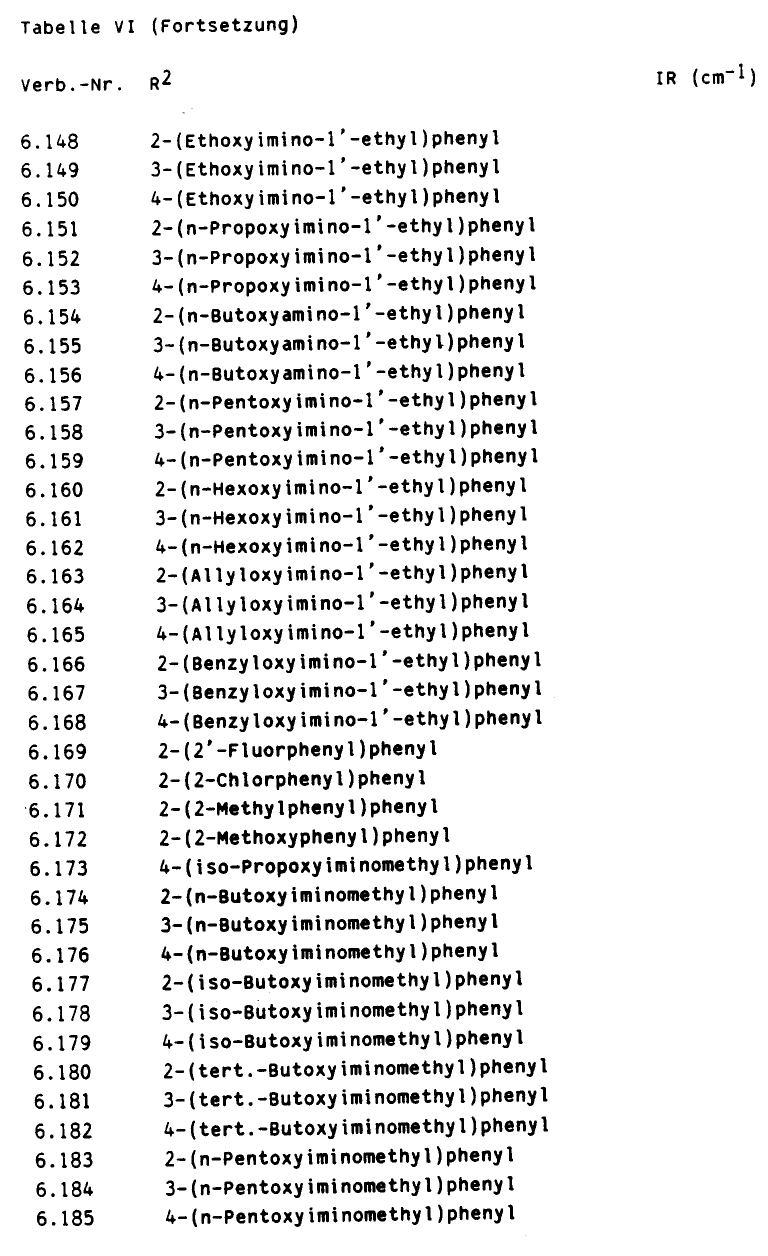

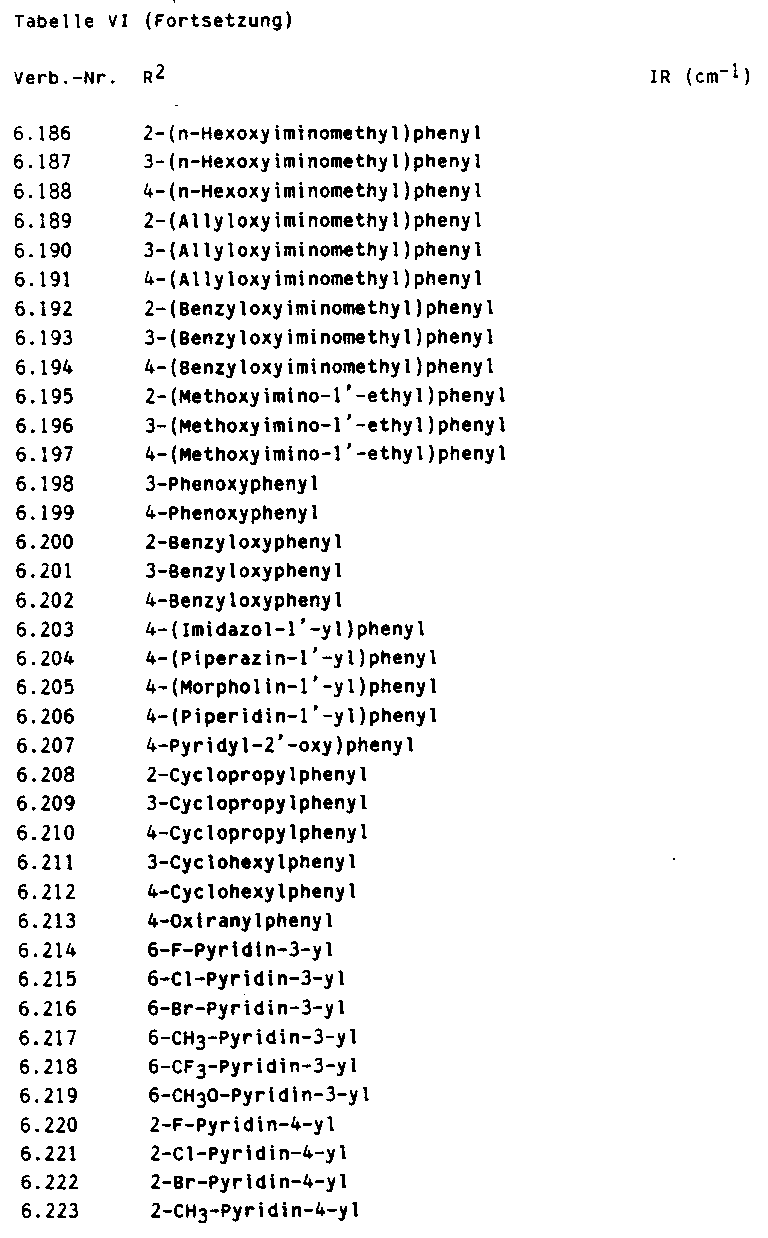

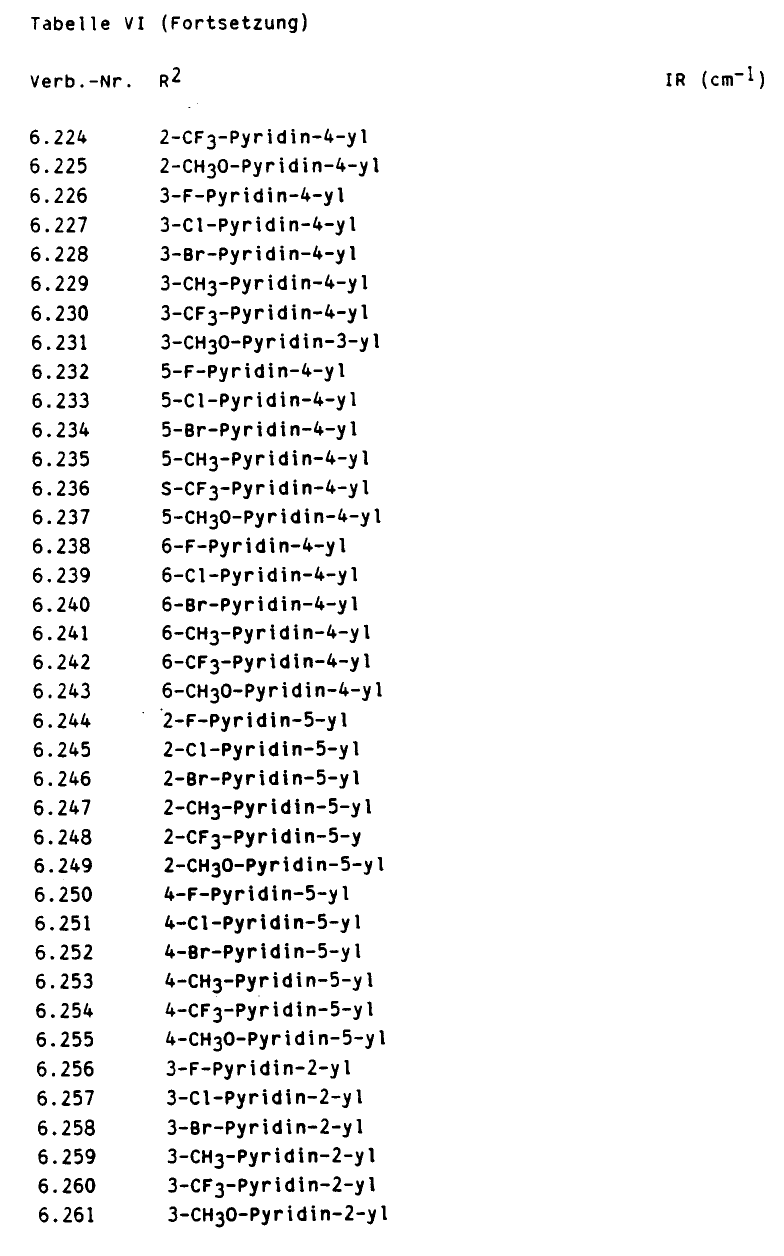

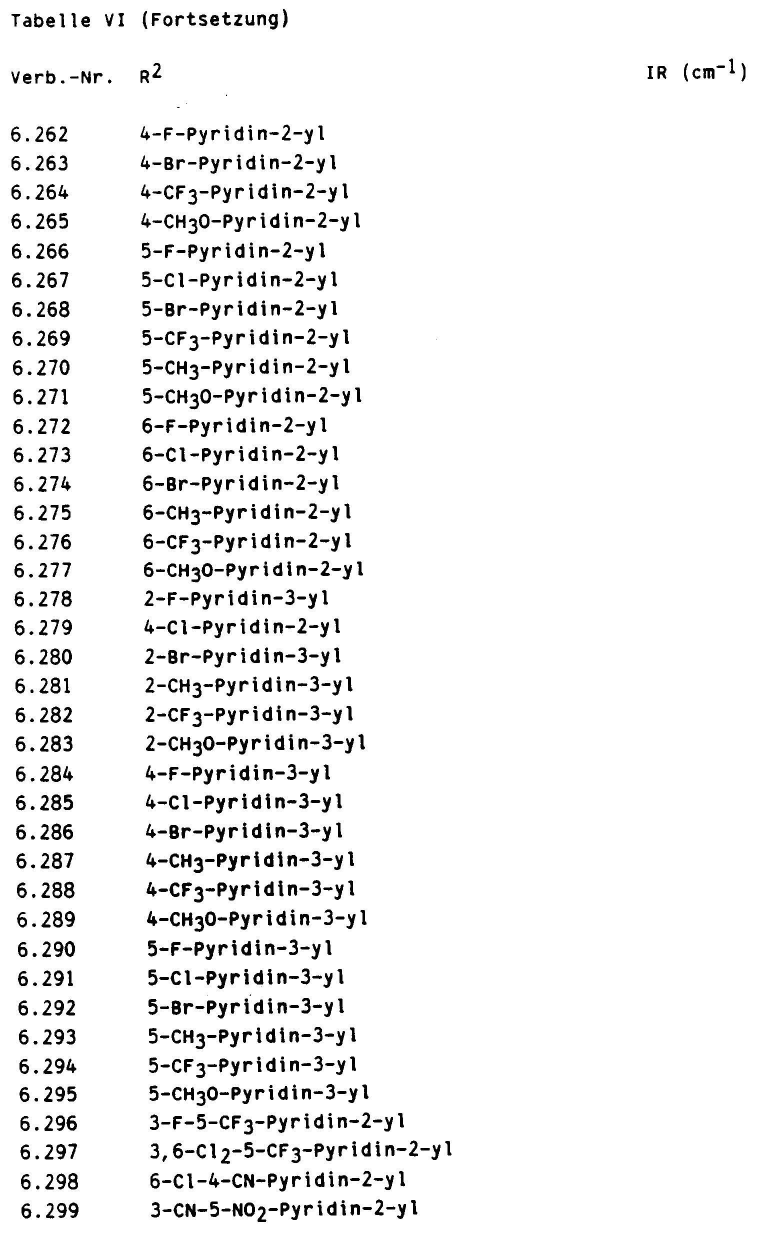

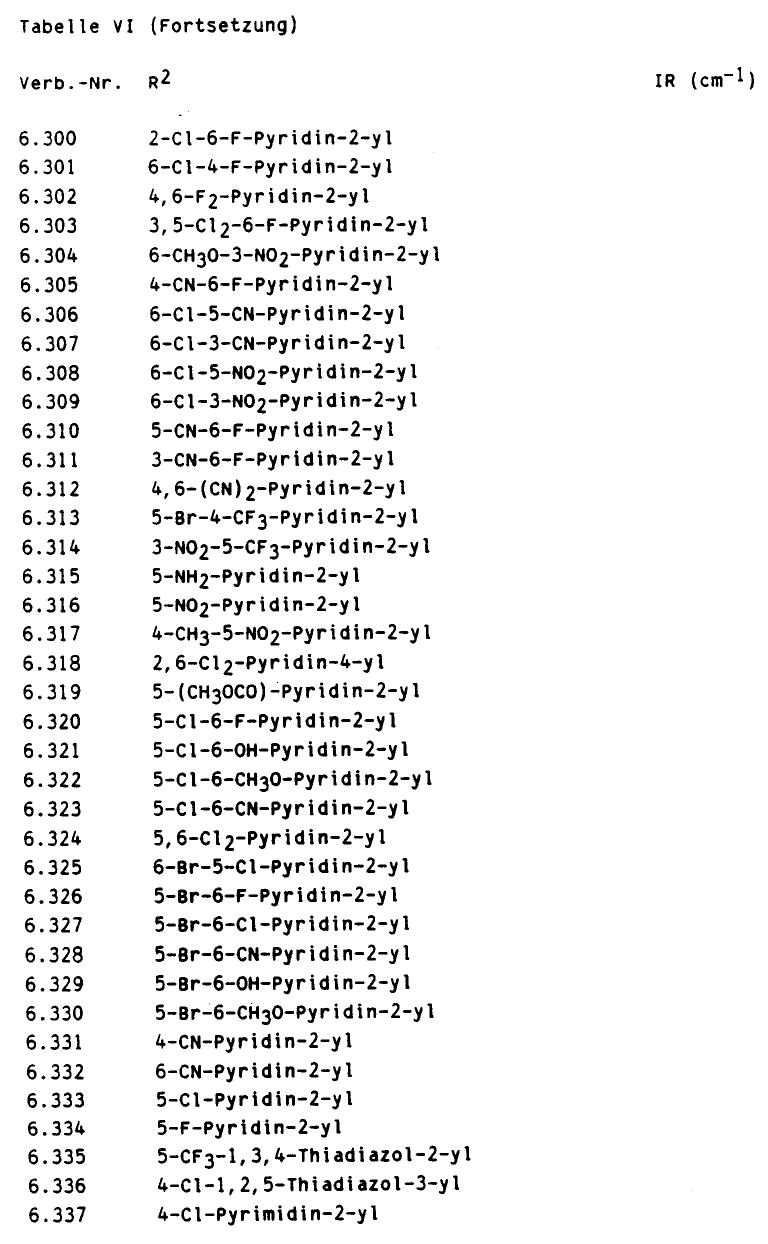

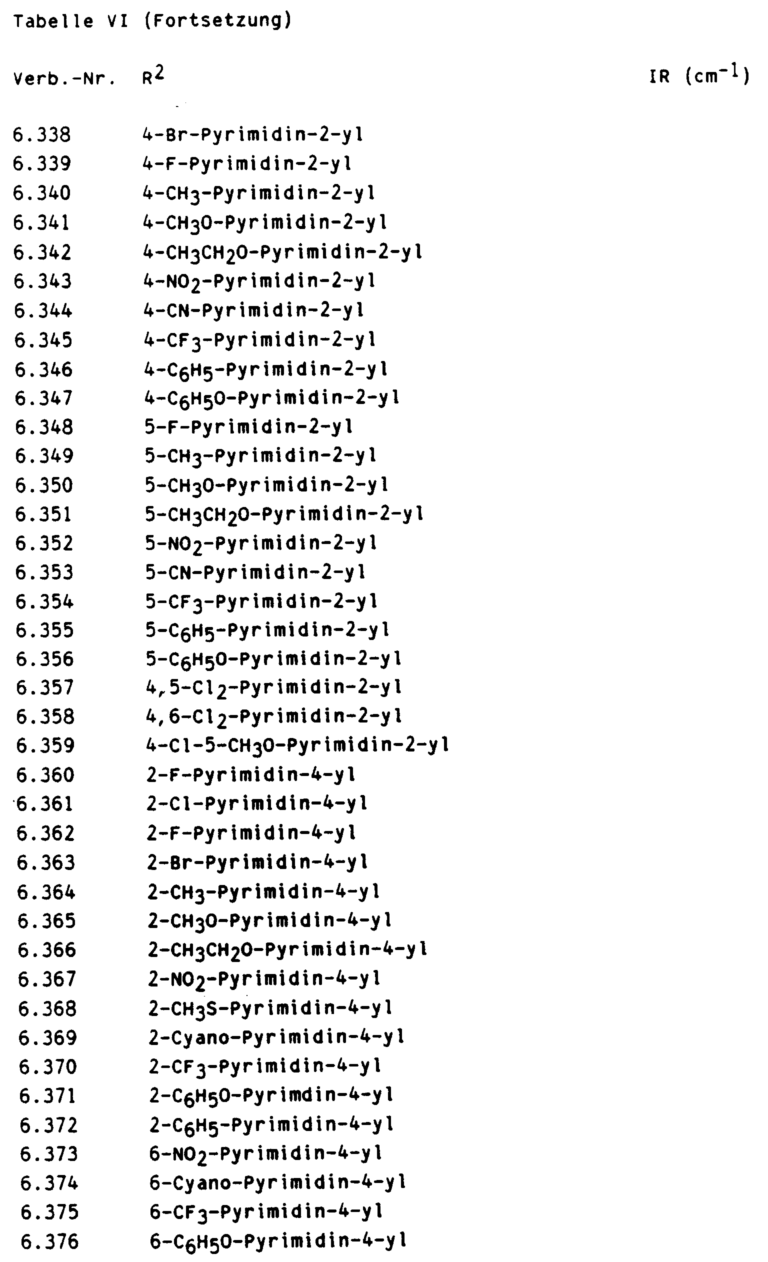

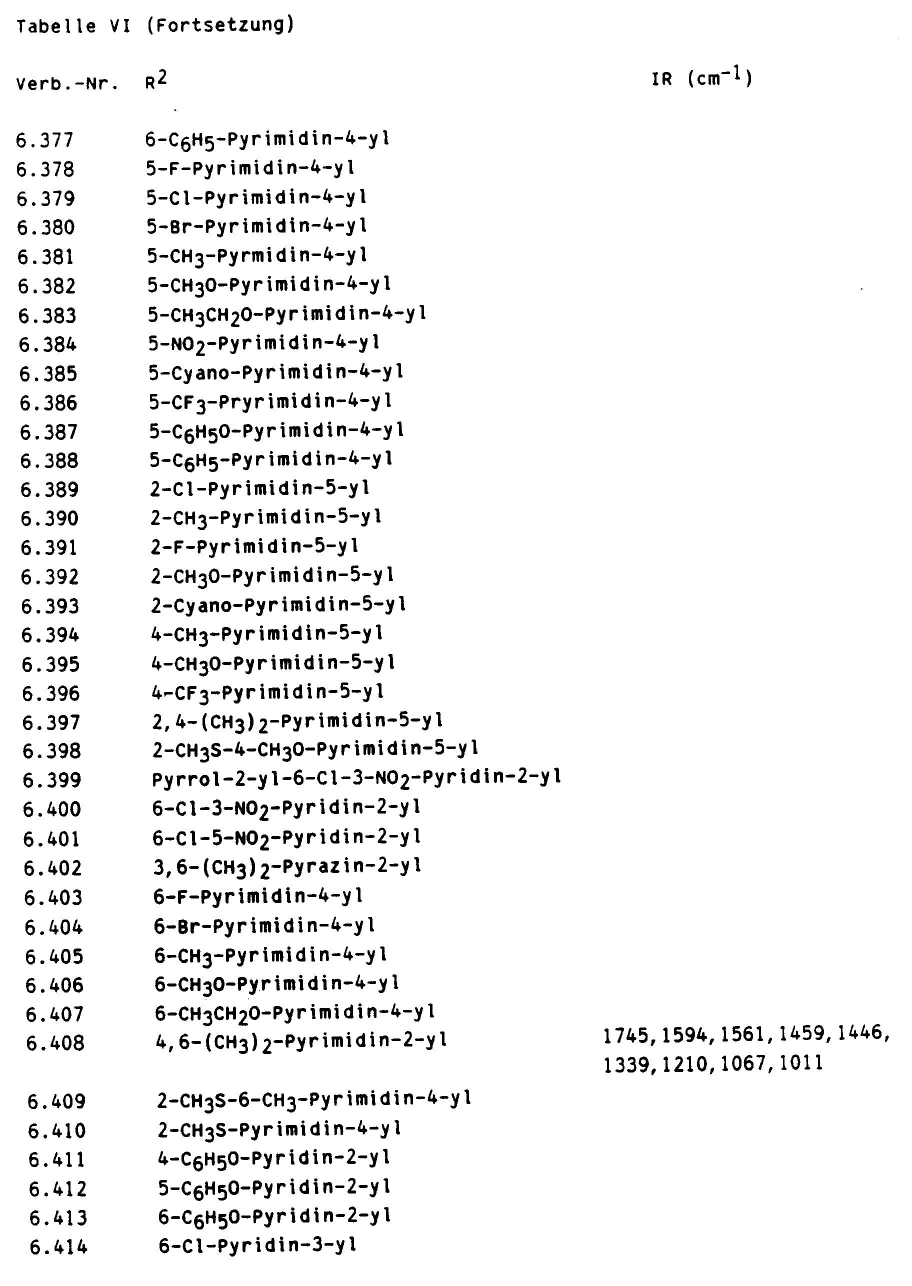

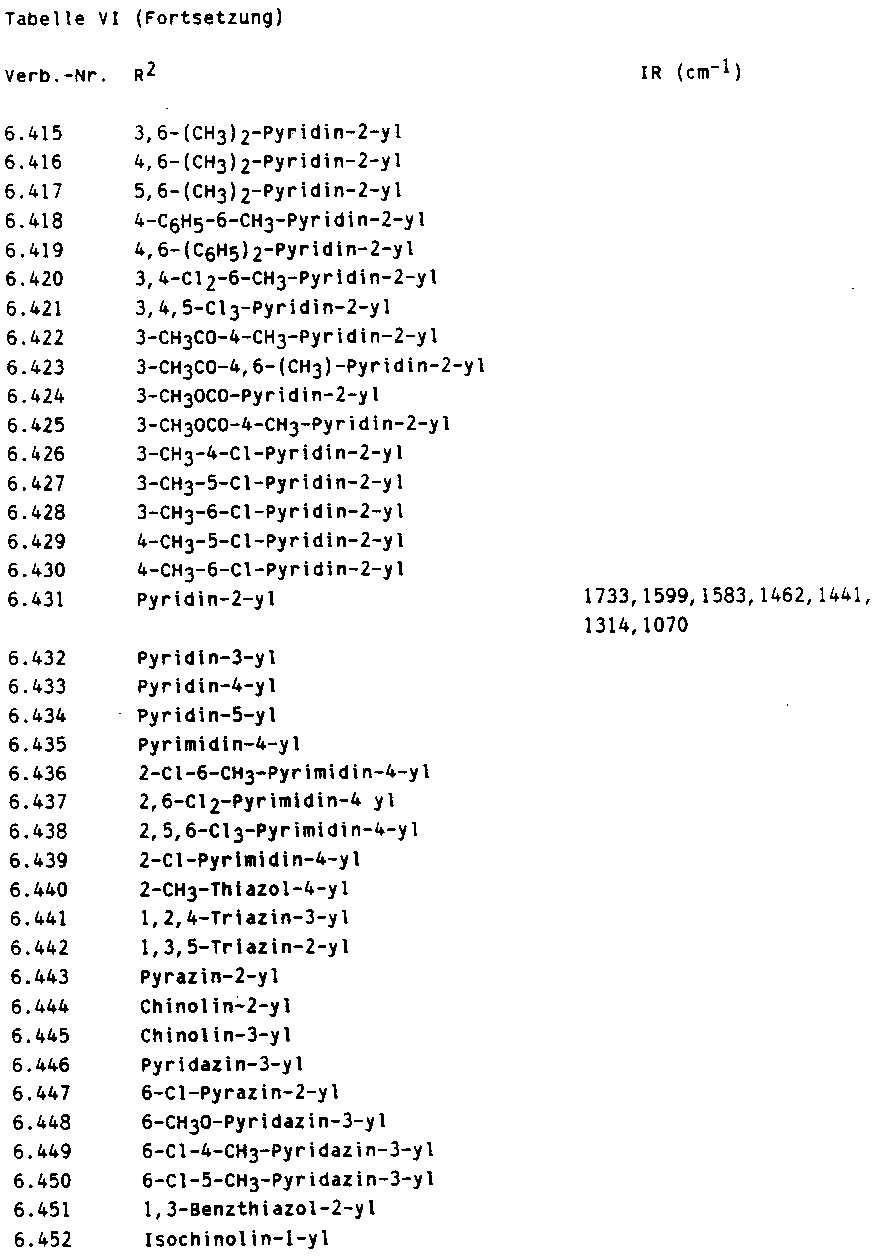

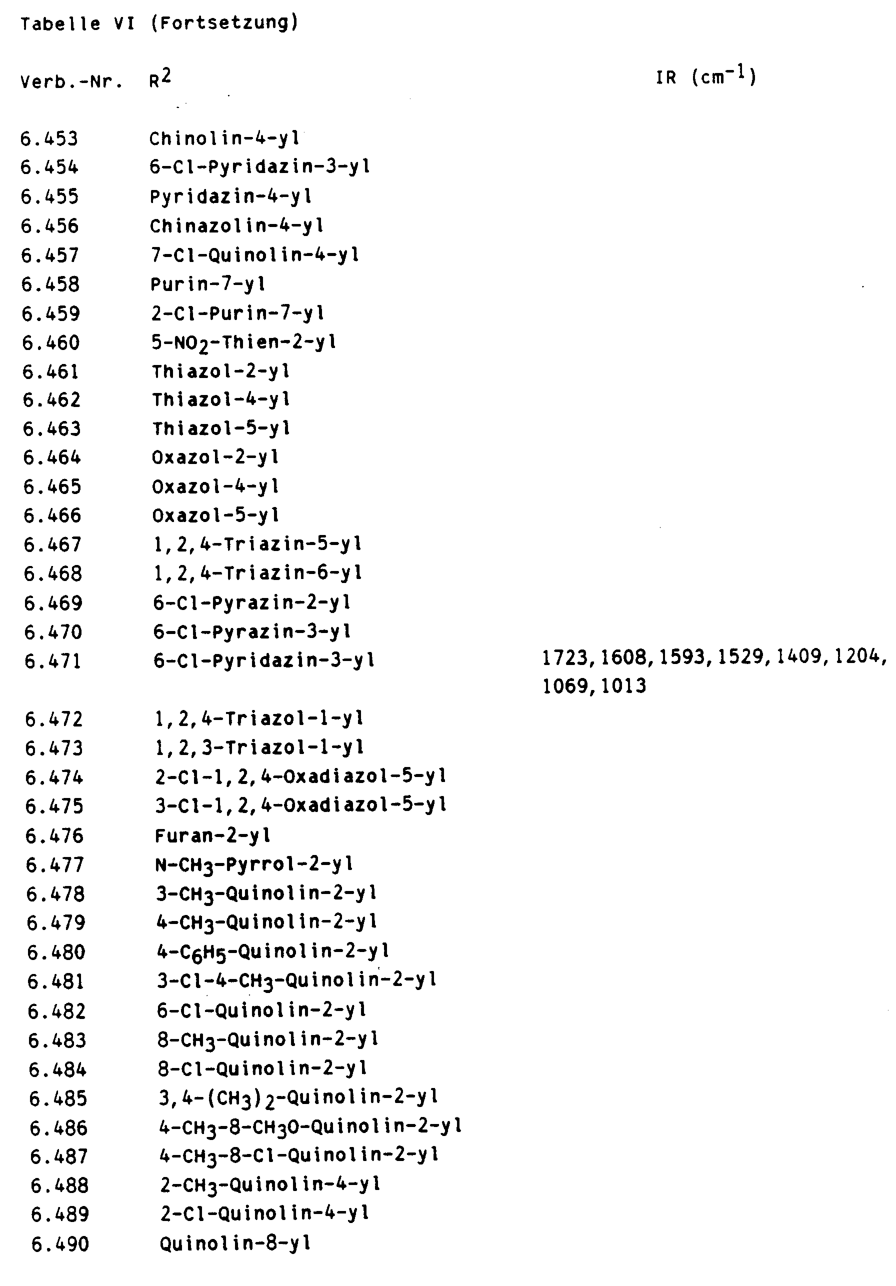

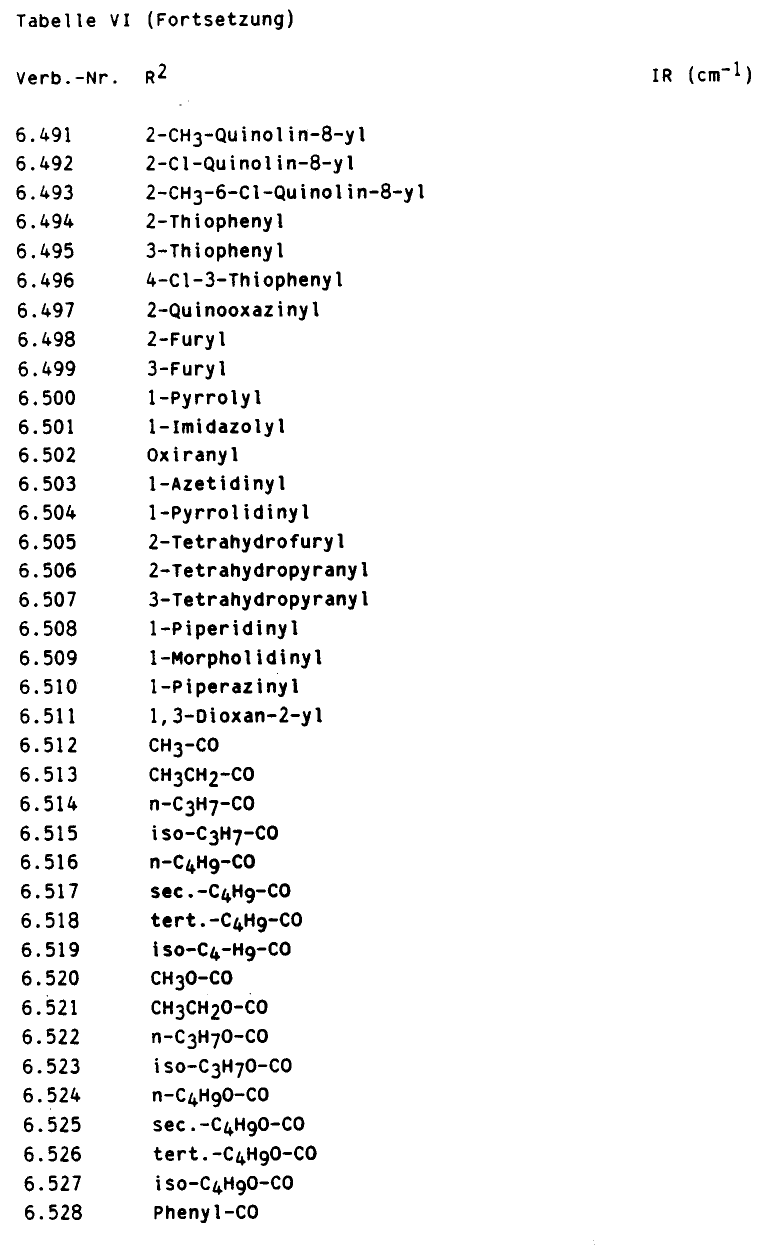

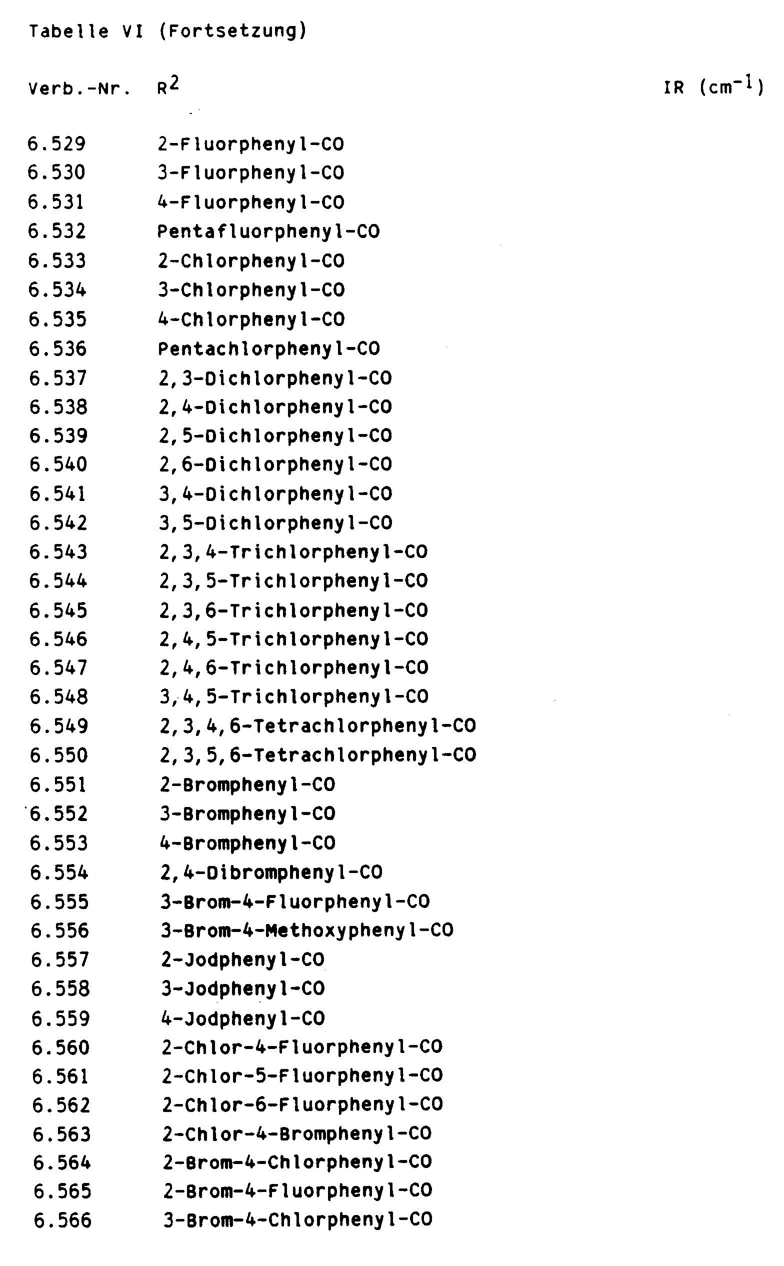

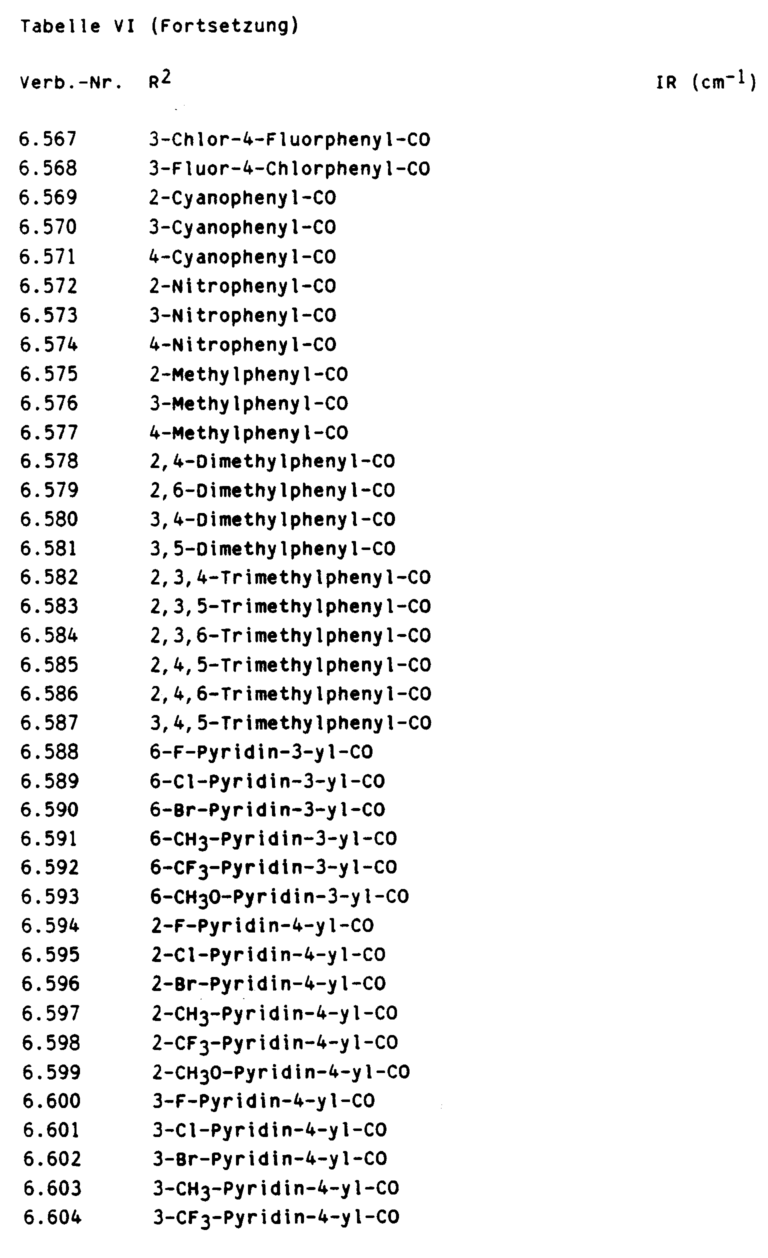

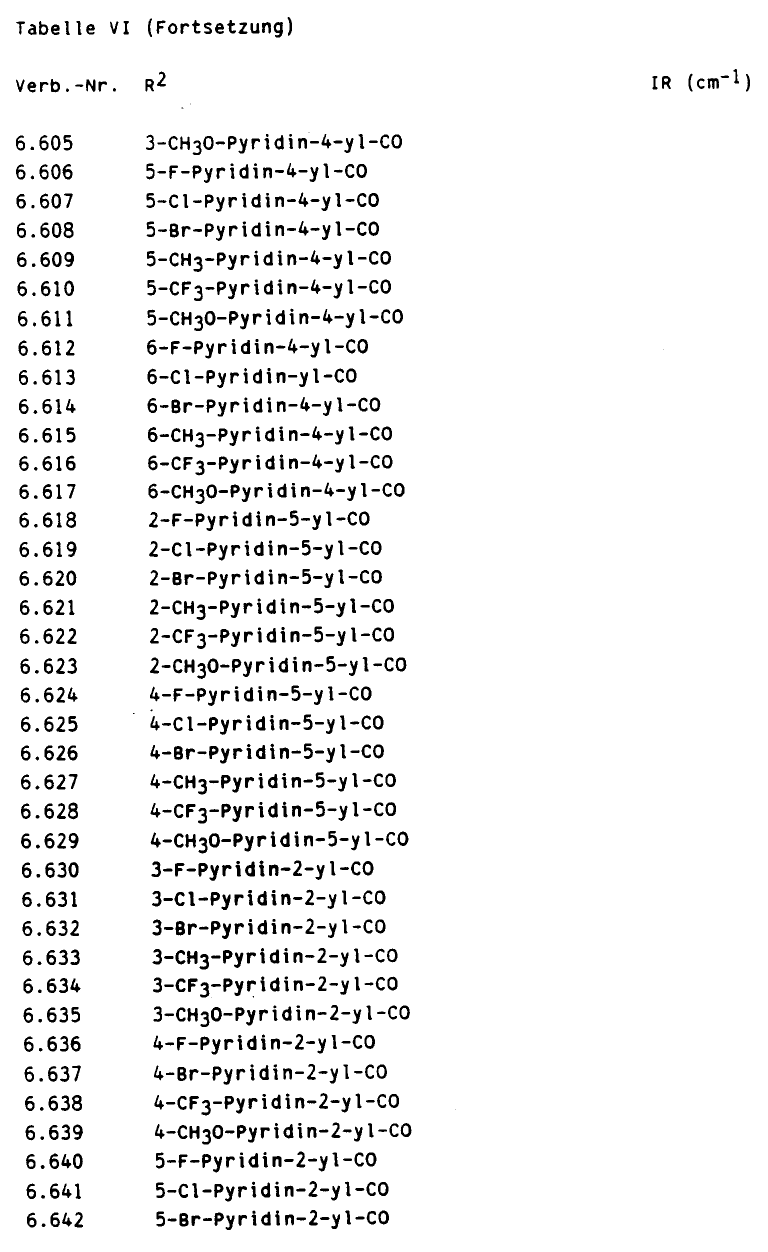

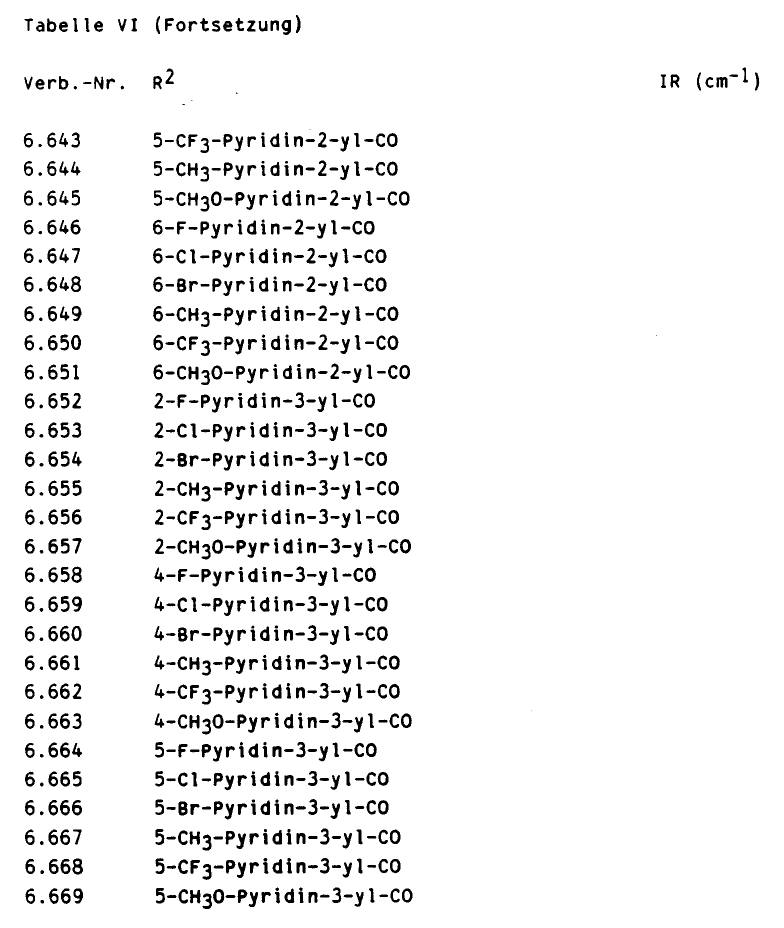

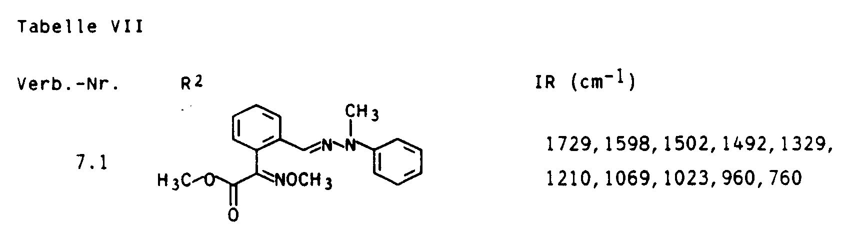

Die neuen Verbindungen eignen sich als Fungizide.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Mußschalenmehl, Cellulosepulver oder andere feste Trägerstoffe.

Beispiele für solche Zubereitungen sind:
I. eine Lösung aus 90 Gew.-Teilen der Verbindung Nr. 1.35 und 10 Gew.-Teilen N-Methyl-α-pyrrolidon, die zur Anwendung in Form kleinster Tropfen geeignet ist;
II. eine Mischung aus 20 Gew.-Teilen der Verbindung Nr. 1.36, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl; durch feines Verteilen der Lösung in Wasser erhält man eine Dispersion.
III. eine wäßrige Dispersion aus 20 Gew.-Teilen der Verbindung Nr. 1.115, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen der Verbindung Nr. 1.341, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen der Verbindung Nr. 1.347, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung Nr. 1.457 und 97 Gew.-Teilen feinteiligem Kaolin; dieses Stäubemittel enthält 3 Gew.-% Wirkstoff;
VII. eine innige Mischung aus 30 Gew.-Teilen der Verbindung Nr. 1.35, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung Nr. 1.36, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. 1.115, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydkondensates und 68 Gew.-Teilen eines paraffinischen Mineralöls.

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester;
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithiolo[4,5-b]chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilid,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

### Anwendungsbeispiele

Als Vergleichswirkstoffe wurden die Verbindung Nr. 4 aus EP 370 629 (A) und Verbindung Nr. 5 aus EP 370 629 (B) benutzt.

### Anwendungsbeispiel 1

### Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß der Wirkstoff 1.35 bei der Anwendung als 0,025 %ige (Gew.%) Spritzbrühe eine bessere fungizide Wirkung zeigt (80 %) als der bekannte Vergleichswirkstoff A (30 %).

### Anwendungsbeispiel 2

### Wirksamkeit gegen Weizenmehltau

Blätter von in Töpfen gewachsenen Weizenkeimlingen der Sorte "Frühgold" wurden mit wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung ermittelt.

Das Ergebnis zeigt, daß der Wirkstoff 1.36 bei der Anwendung als 0,0015 %ige (Gew.%) Spritzbrühe eine bessere fungizide Wirkung zeigt (90 %) als der bekannte Vergleichswirkstoff B (55 %).

### Anwendungsbeispiel 3

### Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

Das Ergebnis zeigt, daß die Wirkstoffe 1.35, 1.36, 1.115, 1.341, 1.347 und 1.457 bei der Anwendung als 0,025 %ige (Gew.%) Spritzbrühe eine sehr gute fungizide Wirkung zeigen (95 %).

## Patentansprüche

1. Verbindungen der Formel I 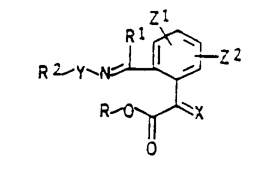 in der die Substituenten folgende Bedeutung haben:
X NOH oder NO-C₁-C₄-alkyl;
R Wasserstoff oder C₁-C₆-Alkyl;
R¹ Wasserstoff;
Z¹,Z² gleich oder verschieden sind und
Wasserstoff, Cyano, Nitro, Halogen, C₁-C₆-Alkyl, C₁-C₄-Halogenalkyl, C₁-C₄-Alkoxy, C₁-C₄-Halogenalkoxy, C₂-C₄-Alkenyl, C₂-C₄-Alkenyloxy, C₂-C₄-Halogenalkenyloxy oder C₂-C₄-Alkinyl bedeuten;
Y Sauerstoff (-O-), Stickstoff (-NH-) oder ein durch C₁-C₆-Alkyl (R³) substituierter Stickstoff (-NR³-);
R² Wasserstoff,
ggf. subst. Alkyl, Cycloalkyl, Alkenyl, Alkinyl oder Cycloalkenyl,
ggf. subst. Aryl, Arylalkyl, Aryloxyalkyl, Arylalkenyl oder Arylcarbonyl,
ggf. subst. Heteroaryl, Heteroarylalkyl, Heteroaryloxyalkyl, Heteroarylalkenyl oder Heteroarylcarbonyl,
ggf. subst. Heterocyclyl oder Heterocyclyloxy,
ggf. subst. Acyl oder
C₁-C₄-Alkoxycarbonyl-C₁-C₄-alkyl,
wobei Y und R³ einen ggf. subst. Ring bilden können.

2. Verbindungen der Formel I gemäß Anspruch 1, in denen Z¹ und Z² unabhängig voneinander für eine der folgenden Gruppen stehen: Halogen, Methyl, Methoxy, Cyano und Nitro.

3. Verbindungen der Formel I gemäß Anspruch 1, in denen Z¹ und Z² Wasserstoff bedeuten.

4. Verbindungen der Formel I gemäß Anspruch 1, in denen R² für eine der folgenden Gruppen steht:
Wasserstoff,
C₁-C₆-Alkyl oder C₁-C₄-Halogenalkyl,
ggf. subst. C₃-C₈-Cycloalkyl oder C₅-C₈-Cycloalkenyl,
ggf. subst. C₃-C₁₅-Alkenyl oder C₃-C₈-Alkinyl,
ggf. subst. Aryl, Aryl-C₁-C₄-alkyl, Aryloxy-C₁-C₄-alkyl, Aryl-C₂-C₄-alkenyl oder Arylcarbonyl
ggf. subst. Heteroaryl, Heteroaryl-C₁-C₄-alkyl, Heteroaryl-C₂-C₄-alkenyl oder Heteroarylcarbonyl,
ggf. subst. Heterocyclyl,
ggf. subst. C₁-C₄-Alkylcarbonyl oder
C₁-C₄-Alkoxycarbonyl-C₁-C₄-alkyl.

5. Verbindung der Formel I gemäß Anspruch 1, dadurch gekennzeichnet, daß R² 2-Methylbenzyl, Y Sauerstoff, R Methyl, Z¹ und Z² Wasserstoff und X NOCH₃ bedeutet.

6. Verbindung der Formel I gemäß Anspruch 1, dadurch gekennzeichnet, daß Z¹ und Z² Wasserstoff, R² 3-Methylbenzyl, Y Sauerstoff, R Methyl und X NOCH₃ bedeutet.

7. 2-(2'-Acetylphenyl)-2-methoxyiminoessigsäuremethylester (III).

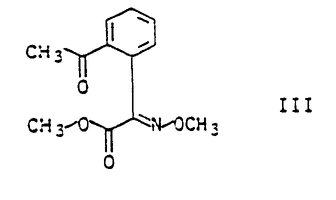

8. Fungizid, enthaltend einen inerten Trägerstoff und eine fungizid wirsame Menge einer Verbindung der allgemeinen Formel I gemäß Anspruch 1.

9. Verfahren zur Bekämpfung von Pilzen, dadurch gekennzeichnet, daß man die Pilze oder die vom Pilzbefall bedrohten Materialien, Pflanzen, Saatgut oder den Erdboden mit einer fungizid wirsamen Menge einer Verbindung der allgemeinen Formel I gemäß Anspruch 1 behandelt.

10. Verfahren zur Herstellung von Verbindungen der Formel I gemäß Anspruch 1, dadurch gekennzeichnet, daß man eine Carbonylverbindung der allgemeinen Formel VII mit einem substituierten Hydroxylamin der Formel XV oder mit einem substituierten Hydrazin der Formel XVI 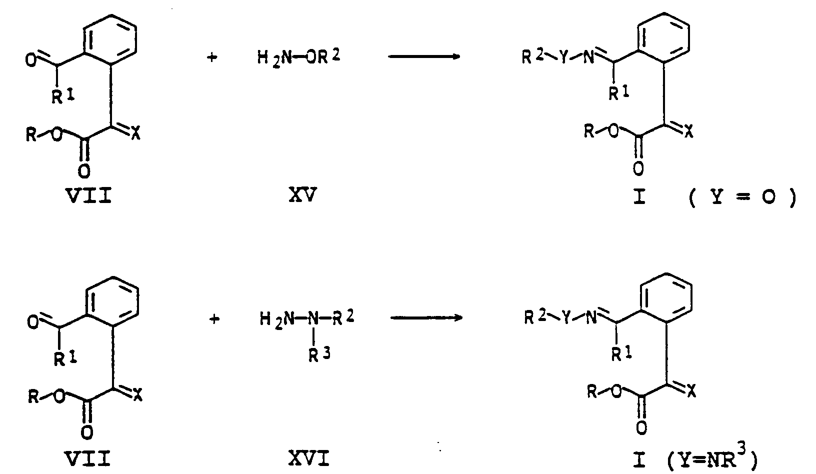 oder mit einem Säureadditionssalz der Verbindungen XV oder XVI umsetzt.

## Claims

1. A compound of the formula I 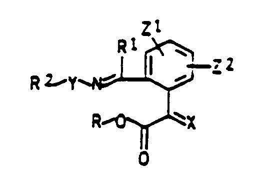 where
X is NOH or NO-C₁-C₄-alkyl;
R is hydrogen or C₁-C₆-alkyl;
R¹ is hydrogen;
Z¹ and Z² are identical or different and each is hydrogen, cyano, nitro, halogen, C₁-C₆-alkyl, C₁-C₄-haloalkyl, C₁-C₄-alkoxy, C₁-C₄-haloalkoxy, C₂-C₄-alkenyl, C₂-C₄-alkenyloxy, C₂-C₄-haloalkenyloxy or C₂-C₄-alkynyl;
Y is oxygen (-O-), nitrogen (-NH-) or C₁-C₆-alkyl (R³)-substituted nitrogen (-NR³-);
R² is hydrogen,
unsubstituted or substituted alkyl, cycloalkyl, alkenyl, alkynyl or cycloalkenyl,
unsubstituted or substituted aryl, arylalkyl, aryloxyalkyl, arylalkenyl or arylcarbonyl,
unsubstituted or substituted hetaryl, hetarylalkyl, hetaryloxyalkyl, hetarylalkenyl or hetarylcarbonyl, unsubstituted or substituted heterocyclyl or heterocyclyloxy,
unsubstituted or substituted acyl or C₁-C₄-alkoxycarbonyl-C₁-C₄-alkyl,
where Y and R³ may form an unsubstituted or substituted ring.

2. A compound of the formula I as claimed in claim 1, where Z¹ and Z² independently of one another are each one of the following groups: halogen, methyl, methoxy, cyano and nitro.

3. A compound of the formula I as claimed in claim 1, where Z¹ and Z² are each hydrogen.

4. A compound of the formula I as claimed in claim 1, where R² is one of the following groups:
hydrogen,
C₁-C₆-alkyl or C₁-C₄-haloalkyl,
unsubstituted or substituted C₃-C₈-cycloalkyl or C₅-C₈-cycloalkenyl,
unsubstituted or substituted C₃-C₁₅-alkenyl or C₃-C₈-alkynyl,
unsubstituted or substituted aryl, aryl-C₁-C₄-alkyl, aryloxy-C₁-C₄-alkyl, aryl-C₂-C₄-alkenyl or arylcarbonyl, unsubstituted or substituted hetaryl, hetaryl-C₁-C₄-alkyl, hetaryl-C₂-C₄-alkenyl or hetarylcarbonyl, unsubstituted or substituted heterocyclyl, unsubstituted or substituted C₁-C₄-alkylcarbonyl or C₁-C₄-alkoxycarhonyl-C₁-C₄-alkyl.

5. A compound of the formula I as claimed in claim 1, wherein R² is 2-methylbenzyl, Y is oxygen, R is methyl, Z¹ and Z² are each hydrogen and X is NOCH₃.

6. A compound of the formula I as claimed in claim 1, wherein Z¹ and Z² are each hydrogen, R² is 3-methylbenzyl, Y is oxygen, R is methyl and X is NOCH₃.

7. Methyl 2-(2'-acetylphenyl)-2-methoxyiminoacetate (III).

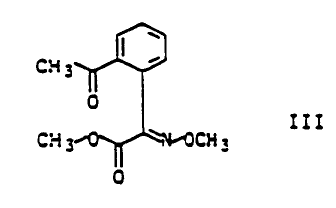

8. A fungicide containing an inert carrier and a fungicidal amount of a compound of the formula I as claimed in claim 1.

9. A method for controlling fungi, which comprises treating the fungi or the materials, plants or seeds threatened by fungal attack or the soil with a fungicidal amount of a compound of the formula I as claimed in claim 1.

10. A process for preparing a compound of the formula I as claimed in claim 1, which comprises reacting a carbonyl compound of the fomula VII with a substituted hydroxylamine of the formula XV or with a substituted hydrazine of the formula XVI 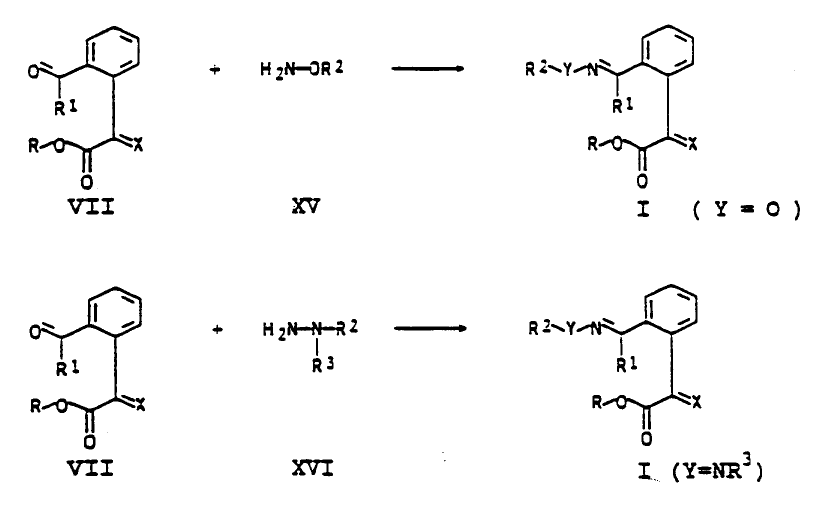 or with an acid addition salt of the compound XV or XVI.

## Revendications

1. Composés de formule I 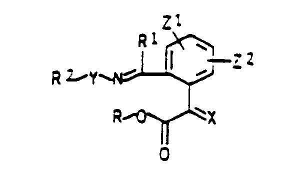 dans laquelle les substituants ont la signification suivante :
X : NOH ou NO-alkyle en C1-C4 ;
R hydrogène ou alkyle en C1-C6
R¹ hydrogène
Z¹, Z², identiques ou différents, représentent hydrogène, cyano, nitro, halogène, alkyle en C1-C6, halogénoalkyle en C1-C4, alcoxy en C1-C4, halogénoalcoxy en C1-C4, alcényle en C2-C4, alcényloxy en C2-C4, halogénoalcényloxy en C2-C4 ou alcinyl en C2-C4 ;
Y oxygène (-O-), azote (-NH-) ou azote substitué par alkyle en C1-C6 (-NR³-) ;
R² hydrogène,
alkyle, cycloalkyle, alcényle, alcinyle ou cycloalcényle éventuellement substitués,
aryle, arylalkyle, aryloxyalkyle, arylalcényle ou arylcarbonyle éventuellement substitués,
hétéroaryle, hétéroarylalkyle, hétéroaryloxyalkyle, hétéroarylalcényle ou hétéroarylcarbonyle éventuellement substitués,
hétérocyclyle ou hétérocyclyloxy éventuellement substitués,
acyle, éventuellement substitué ou
alcoxycarbonyle en C1-C4 -alkyle en C1-C4,
Y et R³ pouvant former un cycle éventuellement substitué.

2. Composés de formule I selon la revendication 1, dans lesquels Z¹ et Z², indépendamment l'un de l'autre, représentent un des groupes suivants : halogène, méthyle, méthoxy, cyano et nitro.

3. Composés de formule I selon la revendication 1, dans lesquels Z¹ et Z² signifient hydrogène.

4. Composés de formule I selon la revendication 1, dans lesquels Z¹ et Z² dans lesquels R² représente un des groupes suivants :
hydrogène,
alkyle en C1-C6 ou halogénoalkyle en C1-C4,
cycloalkyle en C3-C8 ou cycloalcényle en C5-C8, éventuellement substitués,
alcényle en C3-C15 ou alcinyle en C3-C8 éventuellement substitués, aryle, aryle-alkyle en C1-C4 aryloxy-alkyle en C1-C4 aryle-alcényle en C2-C4 ou arylcarbonyle éventuellement substitués,
hétéroaryle, hétéroaryle-alkyle en C1-C4, hétéroaryle-alcényle en C2-C4 ou hétéroarylcarbonyle éventuellement substitués,
hétérocyclyle éventuellement substitué,
alkylcarbonyle en C1-C4 éventuellement substitué ou alcoxycarbonyle en C1-C4-alkyle en C1-C4.

5. Composé de formule I selon la revendication 1, caractérisé par le fait que R² signifie 2-méthylbenzyle, Y oxygène, R méthyle, Z¹ et Z² hydrogène et X NOCH₃.

6. Compose de formule I selon la revendication 1, caractérisé par le fait que Z¹ et Z² signifient hydrogène, R² 3-méthylbenzyle, Y oxygène, R méthyle et X NOCH₃.

7. 2-(2'-acétylphényl)-2-méthoxyiminoacétate de méthyle III

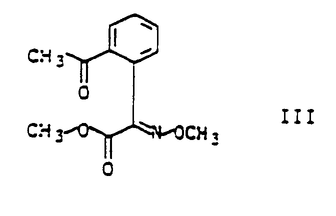

8. Fongicide contenant un véhicule inerte et une quantité à efficacité fongicide d'un composé de formule générale I selon la revendication 1.

9. Procédé de lutte contre les champignons, caractérisé par le fait qu'on traite les champignons ou les matériaux, plantes, semences ou le sol menacés d'une attaque par les champignons avec une quantité à efficacité fongicide d'un composé de formule générale I selon la revendication 1.

10. Procédé de préparation de composés de formule I selon la revendication 1, caractérisé par le fait qu'on convertit un composé carbonyle de formule générale VII avec une hydroxylamine substituée de formule XV ou avec une hydrazine substituée de formule XVI 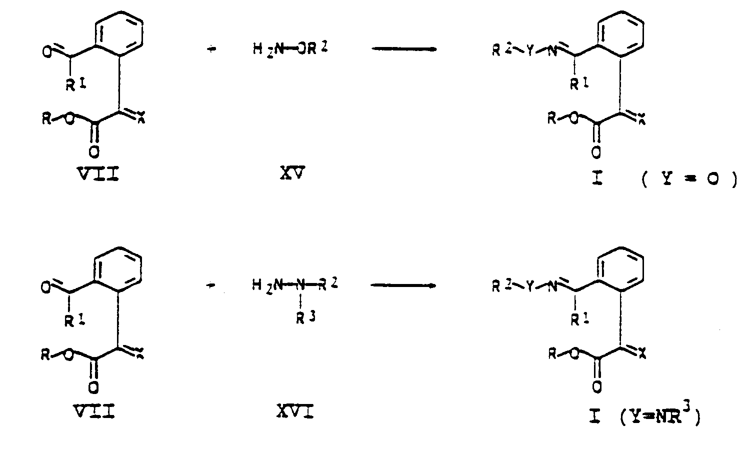 ou avec un sel d'addition d'acide des composés XV ou XVI.
